(19) Europäisches Patentamt
European Patent Office
Office européen des brevets

(11) **EP 3 271 328 B1**

(12) **FASCICULE DE BREVET EUROPEEN**

(45) Date de publication et mention
de la délivrance du brevet:
**19.02.2020 Bulletin 2020/08**

(21) Numéro de dépôt: **16719441.4**

(22) Date de dépôt: **21.03.2016**

(51) Int Cl.:
**C07C 323/52** *(2006.01)*    **C07C 323/58** *(2006.01)*

(86) Numéro de dépôt international:
**PCT/FR2016/050620**

(87) Numéro de publication internationale:
**WO 2016/151229 (29.09.2016 Gazette 2016/39)**

(54) **COMPOSITIONS PULVÉRULENTES D'UN COMPLEXE ENTRE UN ACIDE ET UN MÉTAL À HAUTE TENEUR EN COMPOSÉS ORGANIQUES SOUFRÉS ET LEUR PROCÉDÉ DE PRÉPARATION**

PULVERZUSAMMENSETZUNGEN AUS EINEM KOMPLEX ZWISCHEN EINER SÄURE UND EINEM METALL MIT HOHEM GEHALT AN EINER ORGANOSCHWEFELVERBINDUNG SOWIE VERFAHREN ZUR HERSTELLUNG DAVON

POWDER COMPOSITIONS OF A COMPLEX BETWEEN AN ACID AND A METAL HAVING A HIGH ORGANOSULFUR COMPOUND CONTENT AND METHOD FOR PREPARING SAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.03.2015 FR 1552336**

(43) Date de publication de la demande:
**24.01.2018 Bulletin 2018/04**

(60) Demande divisionnaire:
**19217862.2**

(73) Titulaire: **Innov'ia 3i**
**63380 Pontaumur (FR)**

(72) Inventeurs:
• **BUISSON, Pierre**
  **17140 Lagord (FR)**
• **HUET, Robert**
  **75015 Paris (FR)**
• **FOURNIER, Sébastien**
  **17230 Andilly (FR)**

• **VENDEVILLE, Jean-Eudes**
  **17180 Perigny (FR)**

(74) Mandataire: **Grosset-Fournier, Chantal Catherine et al**
  **Grosset-Fournier & Demachy**
  **54, rue Saint-Lazare**
  **75009 Paris (FR)**

(56) Documents cités:
**EP-A1- 0 140 865      WO-A2-2013/136030**
**DE-A1- 19 707 380     US-A- 4 579 962**

• Khalid Muzaffar ET AL: "Stickiness Problem Associated with Spray Drying of Sugar and Acid Rich Foods: A Mini Review", Nutrition & Food Sciences, 15 June 2015 (2015-06-15), XP055424687, DOI: 10.4172/2155-9600.1000S12003

Remarques:
Le dossier contient des informations techniques présentées postérieurement au dépôt de la demande et ne figurant pas dans le présent fascicule.

**Description**

La présente invention a pour objet des compositions pulvérulentes d'un complexe entre un acide et un métal, à haute teneur en composés organiques soufrés, et leur procédé de préparation.

La méthionine, acide aminé essentiel, et l'acide 2-hydroxy-4-méthyl-thio-butanoïque (HMTBA), un analogue de la méthionine, trouvent des applications largement étendues chez l'homme en tant que complément alimentaire ou médicament, ainsi qu'en nutrition animale. Leurs sels métalliques, par exemple calciques, magnésiques ou de zinc, sous forme solide, sont avantageux car ils permettent de combler des carences en éléments ou oligoéléments. Le sel d'HMTBA le plus connu est le sel dicalcique, comprenant deux moles d'équivalent d'HMTBA par mole de calcium répondant à la formule $(HMTBA)_2Ca$.

De nombreux exemple décrivent la fabrication de sels de méthionine ou d'analogue de la méthionine, notamment le HMTBA.

Dans le cas du HMTBA, si le produit acide concentré se rencontre sous forme liquide, à une concentration élevée souvent supérieure à 87% de HMTBA, les sels sont sous forme solide et sont moins concentrés en HMTBA du fait de l'apport du ou des cations utilisés pour la formation du sel.

Les formes poudres ont pour la plupart une composition correspondant à la stœchiométrie du sel dicalcique $(HMTBA)_2Ca$.

Ainsi, US 4 335 257 décrit la préparation d'un sel de formule $(HMTBA)_2Ca$ qui permet d'obtenir une composition sous forme solide qui n'apporte pas plus de 85% de HMTBA dans sa composition massique.

US 3 272 860, EP 0 049 057, US 6 287 627 et FR 2 964 968 décrivent ainsi l'obtention de sels de formule $(HMTBA)_2Ca$ obtenus à partir d'HMTBA qui sont tous en proportions stœchiométrique et donc à des teneurs en HMTBA maximales de 80 % à 87%.

Peu de documents de l'art antérieur relatent l'obtention de compositions contenant une teneur en HMTBA supérieure à la proportion stœchiométrique de la formation du sel dicalcique $(HMTBA)_2Ca$.

Romoser et al (Poult. Sci. 1976, 55(3), pp 1099-1103) obtiennent une composition riche en MHA sous la forme acide (HMTBA) en pulvérisant ladite forme acide sur un support solide (vermiculite). Mais la teneur en HMTBA n'est dans ce cas que de 50%.

De même, EP 140865 décrit l'obtention de sels de calcium d'HMTBA consistant en plus de deux et moins de dix moles d'équivalent d'HMTBA par mole de calcium. Ces sels sont obtenus en mettant en réaction l'HMTBA avec une source de calcium choisie parmi l'oxyde calcium (CaO), l'hydroxyde de calcium $(Ca(OH)_2)$, le carbonate de calcium $(CaCO_3)$ ainsi qu'un sel d'HMTBA, par exemple le sel $(HMTBA)_2$ Ca. L'HMTBA est généralement en solution aqueuse fortement concentrée, à laquelle la source de calcium est mélangée, puis le milieu réactionnel ainsi obtenu est séché à une température de l'ordre de 70°C.

Le produit obtenu est à 94% d'équivalent HMTBA. Cependant le milieu réactionnel d'HMTBA avec la source de calcium est très visqueux et collant: il est donc très difficile à homogénéiser dans des mélangeurs ou réacteurs équipés de systèmes d'agitation classiques et, en fin de réaction, il est nécessaire de procéder à un séchage in-situ pour pouvoir vidanger le réacteur. Elle nécessite de plus une étape de mise en forme après séchage par broyage puis tamisage. Enfin, ce procédé, obligatoirement par batch, ne permet pas une adaptation de procédé en continu.

Un recyclage de sel de calcium d'HMTBA, par exemple de sel $(HMTBA)_2Ca$, à la source de calcium avant d'ajouter l'HMTBA, permet d'améliorer la consistance du milieu réactionnel et facilite la mise en œuvre du procédé. Mais comme décrit dans US 4 335 257 cette amélioration nécessite au moins 20% d'apport pondéral dudit sel et jusqu'à 80% dudit sel. De part cette nécessité, ce procédé réduit la productivité et augmente le surdimensionnement des installations.

Aussi, un but de la présente invention consiste à fournir des complexes entre un acide et un métal sous forme solide de poudre ayant une teneur en composés organiques soufrés, en particulier en HMTBA, supérieure à 87%.

Un autre but de l'invention consiste à fournir, des complexes entre un acide et un métal, sous la forme de poudres stables, aisément manipulables et adaptées à l'application à laquelle lesdits complexes sont destinés.

Un autre but de l'invention consiste à fournir un procédé de préparation de complexes entre un acide et un métal, en batch, sans difficultés de collage ou apparition de masse visqueuse lors de la préparation, et sans utilisation de pied de cuve pour réaliser le produit.

Un autre but de l'invention consiste à fournir un procédé de préparation de complexes entre un acide et un métal, en continu.

La description a par conséquent pour objet une particule comprenant :

• un noyau constitué essentiellement d'un sel de formule (I) suivante :

$$(A^-)_n M^{n+} \qquad (I)$$

dans laquelle :

A⁻ représente un anion choisi dans le groupe constitué du 2-hydroxy-4-méthyl-thio-butanoate, du méthioniate et du cystéinate,

M représente un métal divalent ou trivalent,

n étant égal à 2 lorsque ledit métal est divalent et à 3 lorsque ledit métal est trivalent, et

- une couche comprenant un composé B choisi dans le groupe constitué de l'acide 2-hydroxy-4-méthyl-thio-butanoïque (HMTBA), de la méthionine, de la cystéine, de leurs mélanges, de leurs sels et de leurs complexes,

ladite couche enrobant ledit noyau,

le pourcentage massique dudit composé B par rapport au sel de formule (I) du noyau étant compris d'environ 10% à environ 50%,

ledit composé B n'étant pas, ou pas uniquement, sous la forme d'un sel de formule (I),

la teneur en composés organiques soufrés (TOS) de ladite particule étant supérieure à 87% en masse, en particulier supérieure à 88%, 89% ou 90% en masse, par rapport à la masse totale de ladite particule.

L'invention a par conséquent pour objet une particule comprenant :

- un noyau constitué essentiellement d'un sel de formule (I) suivante :

$$(A^-)_n M^{n+} \qquad (I)$$

dans laquelle :

A⁻ représente un anion choisi dans le groupe constitué du 2-hydroxy-4-méthyl-thio-butanoate, du méthioniate et du cystéinate,

M représente un métal divalent ou trivalent,

n étant égal à 2 lorsque ledit métal est divalent et à 3 lorsque ledit métal est trivalent, et

- une couche comprenant un composé B choisi dans le groupe constitué de l'acide 2-hydroxy-4-méthyl-thio-butanoïque (HMTBA), de ses sels et de ses complexes, et de leurs mélanges.

ladite couche enrobant ledit noyau.

le pourcentage massique dudit composé B par rapport au sel de formule (I) du noyau étant compris de 10% à 40%.

ledit composé B n'étant pas, ou pas uniquement, sous la forme d'un sel de formule (I),

la teneur en composés organiques soufrés (TOS) de ladite particule étant supérieure à 87% en masse, en particulier supérieure à 88%. 89% ou 90% en masse, par rapport à la masse totale de ladite particule.

La teneur en composés organiques soufrés correspond au TOS (Total Organic Sulfur).

Lorsque la particule comprend du HMTBA, la teneur en HMTBA est mesurée par mesure du TOS.

De façon surprenante, la particule obtenue est stable et reste sous forme pulvérulente, malgré des TOS élevés. Elle n'est ni visqueuse ni collante.

Par particule, on entend un petit élément de matière qui, à l'œil nu, apparaît d'un seul tenant et non constitué d'une juxtaposition d'éléments plus petits.

Par « noyau constitué essentiellement d'un sel de formule (I) », on entend en particulier un noyau comprenant plus de 70% en masse dudit sel de formule (I).

Le pourcentage massique dudit composé B compris dans ladite couche est donné par rapport à la masse de sel de formule (I) du noyau, et non par rapport à la masse totale de ladite particule.

Par « ledit composé B n'est pas, ou pas uniquement, sous la forme d'un sel de formule (I) », on entend que le composé B est sous une forme autre qu'un sel de formule (I), par exemple sous forme libre ou sous forme d'un complexe de formule (II) telle que décrite ci-après, ou sous la forme d'un mélange comprenant un sel de formule (I) et au moins une autre forme telle que la forme libre ou un complexe de formule (II).

Selon un mode de réalisation avantageux, la description décrit une particule telle que définie précédemment, dans laquelle ledit composé B est sous la forme :

- libre, choisie parmi l'acide 2-hydroxy-4-méthyl-thio-butanoïque (HMTBA), la méthionine et la cystéine, et/ou
- dudit sel de formule (I) tel que défini précédemment, et/ou
- d'un complexe de formule $(A)_4 M$ (II) dans laquelle A et M sont tels que définis précédemment, A représentant de préférence l'acide 2-hydroxy-4-méthyl-thio-butanoïque (HMTBA),

ledit composé B n'étant pas, ou pas uniquement, sous la forme d'un sel de formule (I),

ledit composé B étant en particulier sous la forme :

- libre,
- du complexe de formule (II),
- d'un mélange de la forme libre et du complexe de formule (II),
- d'un mélange de la forme libre et du sel de formule (I),
- d'un mélange du sel de formule (I) et du complexe de formule (II), ou
- d'un mélange de la forme libre, du sel de formule (I) et du complexe de formule (II).

Selon un mode de réalisation avantageux, présente invention concerne une particule telle que définie précédemment, dans laquelle ledit composé B est sous la forme :

- libre, choisie parmi l'acide 2-hydroxy-4-méthyl-thio-butanoïque (HMTBA), et/ou
- dudit sel de formule (I) tel que défini dans la revendication 1. et/ou
- d'un complexe de formule $(A)_4M$ (II) dans laquelle A et M sont tels que définis dans la revendication 1. A représentant l'acide 2-hydroxy-4-méthyl-thio-butanoïque (HMTBA),

ledit composé B n'étant pas, ou pas uniquement, sous la forme d'un sel de formule (I),
ledit composé B étant en particulier sous la forme :

- libre.
- du complexe de formule (II),
- d'un mélange de la forme libre et du complexe de formule (II),
- d'un mélange de la forme libre et du sel de formule (I),
- d'un mélange du sel de formule (I) et du complexe de formule (II), ou
- d'un mélange de la forme libre, du sel de formule (I) et du complexe de formule (II).

Selon un mode de réalisation avantageux, la présente invention concerne une particule telle que définie précédemment, dans laquelle ledit composé B est sous la forme :

- du complexe de formule (II),
- d'un mélange de la forme libre et du complexe de formule (II),
- d'un mélange du sel de formule (I) et du complexe de formule (II), ou
- d'un mélange de la forme libre, du sel de formule (I) et du complexe de formule (II),

ledit composé B étant en particulier sous la forme du complexe de formule (II).
Selon un mode de réalisation avantageux, la présente invention concerne une particule telle que définie précédemment, comprenant moins de 3%, en particulier moins de 2% ou 1,5%, d'eau en masse.
Selon un mode de réalisation avantageux, la présente invention concerne une particule telle que définie précédemment, dont la teneur en calcium est comprise de 6 à 11% en masse, en particulier de 6,5 à 10%, plus particulièrement de 7 à 9%, encore plus particulièrement de 7,5%, 8,0% ou 8,5%, la teneur en calcium étant notamment d'environ 8%.
Selon un mode de réalisation avantageux, la présente invention concerne une particule telle que définie précédemment, dans laquelle le pourcentage massique dudit composé B par rapport au sel de formule (I) du noyau est compris d'environ 10% à environ 40%, en particulier d'environ 15 % à environ 35%, plus particulièrement d'environ 20% à environ 32%, encore plus particulièrement de 21%, 22%, 23%, 24%, 25%, 26%, 27%, 28%, 29%, 30% ou 31%.
Selon un mode de réalisation avantageux, la présente invention concerne une particule telle que définie précédemment, dans laquelle ledit métal est choisi dans le groupe comprenant Mg, Be, Ca, Sr, Ba, Mn, Fe, Co, Ni, Cu, Zn, Pt, B, Al, Ga, In, en particulier Mg, Ca, Fe, Mn, Al, Cu, Zn.
Selon un mode de réalisation avantageux, la présente invention concerne une particule telle que définie précédemment, dans laquelle ledit métal et n sont tels que $M^{n+}$ représente $Mg^{2+}$, $Be^{2+}$, $Ca^{2+}$, $Sr^{2+}$, $Ba^{2+}$, $Mn^{2+}$, $Fe^{2+}$, $Fe^{3+}$, $Co^{2+}$, $Co^{3+}$, $Ni^{2+}$, $Ni^{3+}$, $Cu^{2+}$, $Zn^{2+}$, $Pt^{2+}$, $Al^{3+}$, $Ga^{3+}$ ou $In^{3+}$, en particulier $Mg^{2+}$, $Ca^{2+}$, $Fe^{2+}$, $Fe^{3+}$, $Al^{3+}$, $Mn^{2+}$, $Cu^{2+}$ ou $Zn^{2+}$.
Selon un mode de réalisation avantageux, la présente invention concerne une particule telle que définie précédemment, dans laquelle ledit sel de formule (I) est $(HMTBA)_2Ca$, $(HMTBA)_2Mg$, $(HMTBA)_2Fe$, $(HMTBA)_2Mn$, $(HMTBA)_2Zn$, $(HMTBA)_2Cu$, $(HMTBA)_3Fe$, $(HMTBA)_3Al$, $(Méthionine)_2Ca$, $(Méthionine)_2Mg$, $(Méthionine)_2Fe$, $(Méthionine)_2Mn$, $(Méthionine)_2Zn$, $(Méthionine)_2Cu$, $(Méthionine)_3Fe$, $(Méthionine)_3Al$, $(Cystéine)_2Ca$, $(Cystéine)_2Mg$, $(Cystéine)_2Fe$, $(Cystéine)_2Mn$, $(Cystéine)_2Zn$, $(Cystéine)_2Cu$, $(Cystéine)_3Fe$, ou $(Cystéine)_3Al$, encore plus particulièrement un sel de formule $(HMTBA)_2Ca$, $(HMTBA)_2Mg$, $(HMTBA)_2Fe$, $(HMTBA)_2Mn$, $(HMTBA)_2Zn$, $(HMTBA)_2Cu$, $(Méthionine)_2Ca$,

(Méthionine)$_2$Mg, (Méthionine)$_2$Fe, (Méthionine)$_2$Mn, (Méthionine)$_2$Zn, (Méthionine)$_2$Cu, (Cystéine)$_2$Ca, (Cystéine)$_2$Mg, (Cystéine)$_2$Fe, (Cystéine)$_2$Mn, (Cystéine)$_2$Zn ou (Cystéine)$_2$Cu.

Selon un mode de réalisation avantageux, la présente invention concerne une particule telle que définie précédemment, dans laquelle ledit anion A$^-$ est le 2-hydroxy-4-méthyl-thio-butanoate.

Selon un mode de réalisation avantageux, la présente invention concerne une particule telle que définie précédemment, dans laquelle ledit composé B compris dans ladite couche est l'acide 2-hydroxy-4-méthyl-thio-butanoïque (HMTBA), ou l'un de ses sels ou complexes.

Selon un mode de réalisation avantageux, la présente invention concerne une particule telle que définie précédemment, dans laquelle ledit anion A$^-$ est le 2-hydroxy-4-méthyl-thio-butanoate, et ledit composé B compris dans ladite couche est l'acide 2-hydroxy-4-méthyl-thio-butanoïque (HMTBA), ou l'un de ses sels ou complexes.

Selon un mode de réalisation avantageux, la présente invention concerne une particule telle que définie précédemment, dans laquelle le composé A formant ledit anion A$^-$ et ledit composé B si B est sous forme libre, ou le composé formant le composé B, si B est sous forme de sel ou de complexe, sont différents.

Selon un mode de réalisation avantageux, la présente invention concerne une particule telle que définie précédemment, dans laquelle ledit acide 2-hydroxy-4-méthyl-thio-butanoïque (HMTBA) est sous forme monomérique à plus de 60% en masse.

Selon un mode de réalisation avantageux, la présente description décrit une particule comprenant :

- un noyau constitué essentiellement de sel de formule (I) (HMTBA)$_2$Ca, et
- une couche comprenant un composé B choisi dans le groupe constitué de l'acide 2-hydroxy-4-méthyl-thio-butanoïque (HMTBA), de la méthionine, de la cystéine, de leurs mélanges, de leurs sels et de leurs complexes,

ladite couche enrobant ledit noyau,
le pourcentage massique dudit composé B par rapport au sel de formule (I) (HMTBA)$_2$Ca du noyau étant compris d'environ 10% à environ 50%,
ledit composé B n'étant pas, ou pas uniquement, sous la forme du sel de formule (I) (HMTBA)$_2$Ca,
la teneur en composés organiques soufrés (TOS) de ladite particule étant supérieure à 87% en masse, en particulier supérieure à 88%, 89% ou 90% en masse, par rapport à la masse totale de ladite particule.

Selon un mode de réalisation avantageux, la présente description décrit une particule comprenant :

- un noyau constitué essentiellement de sel de formule (I) (HMTBA)$_2$Ca, et
- une couche comprenant de l'acide 2-hydroxy-4-méthyl-thio-butanoïque (HMTBA),

ladite couche enrobant ledit noyau,
le pourcentage massique du HMTBA de ladite couche par rapport au sel de formule (I) (HMTBA)$_2$Ca du noyau étant compris d'environ 10% à environ 50%,
le HMTBA de ladite couche n'étant pas, ou pas uniquement, sous la forme du sel de formule (I) (HMTBA)$_2$Ca,
la teneur en composés organiques soufrés (TOS) de ladite particule étant supérieure à 87% en masse, en particulier supérieure à 88%, 89% ou 90% en masse, par rapport à la masse totale de ladite particule.

Selon un mode de réalisation avantageux, la présente description décrit une particule comprenant :

- un noyau constitué essentiellement de sel de formule (I) (HMTBA)$_2$Ca, et
- une couche comprenant de l'acide 2-hydroxy-4-méthyl-thio-butanoïque (HMTBA) et du calcium, le rapport atomique S/Ca étant compris de 2,7 à 3,7,

ladite couche enrobant ledit noyau,
le pourcentage massique du HMTBA de ladite couche par rapport au sel de formule (I) (HMTBA)$_2$Ca du noyau étant compris d'environ 10% à environ 50%,
la teneur en composés organiques soufrés (TOS) de ladite particule étant supérieure à 87% en masse, en particulier supérieure à 88%, 89% ou 90% en masse, par rapport à la masse totale de ladite particule.

Selon un mode de réalisation avantageux, la présente description décrit une particule comprenant :

- un noyau constitué essentiellement de sel de formule (I) (HMTBA)$_2$Ca, et
- une couche comprenant du complexe de formule (II) (HMTBA)$_4$Ca,

ladite couche enrobant ledit noyau,
le pourcentage massique du HMTBA de ladite couche par rapport au sel de formule (I) (HMTBA)$_2$Ca du noyau étant compris d'environ 10% à environ 50%,

la teneur en composés organiques soufrés (TOS) de ladite particule étant supérieure à 87% en masse, en particulier supérieure à 88%, 89% ou 90% en masse, par rapport à la masse totale de ladite particule.

L'invention concerne également une composition pulvérulente constituée ou comprenant des particules telles que définies précédemment.

Par « composition pulvérulente de particules », on entend un solide, dans un état fractionné, c'est-à-dire constitué de particules, selon la présente invention.

Selon un mode de réalisation avantageux, la présente invention concerne une composition telle que définie précédemment, dans laquelle la taille granulométrique desdites particules varie de 10 à 3000 $\mu$m, en particulier de 20 à 300 $\mu$m, plus particulièrement de 100 à 250 $\mu$m, en moyenne granulométrique [Dv(0,5)].

Par moyenne granulométrique [Dv(0,5)], on entend le diamètre granulométrique moyen, mesuré par diffraction laser, 50% des particules de ladite composition ayant un diamètre supérieur au dit diamètre moyen et 50% des particules de ladite composition ayant un diamètre inférieur au dit diamètre moyen.

Selon un mode de réalisation avantageux, la présente invention concerne une composition telle que définie précédemment, dont la masse volumique non tassée est supérieure à 350 g/L, en particulier supérieure à 400 g/L.

La masse volumique non tassée (ou aérée) de ladite composition pulvérulente peut être mesurée à l'aide d'une éprouvette de 250 ml graduée tous les 2 ml. Cette méthode est décrite dans la norme AFNOR NF X 04-344. Le mode opératoire consiste à verser la composition pulvérulente dans l'éprouvette de manière à se situer proche de la graduation maximum du récipient et ensuite mesurer la masse ainsi que le volume occupé par ladite composition pulvérulente. La masse volumique non tassée est ensuite calculée par le rapport de la masse de poudre sur le volume occupé par la composition pulvérulente.

Selon un mode de réalisation avantageux, la présente invention concerne une composition telle que définie précédemment, dont la masse volumique tassée après 10 coups est supérieure à 400 g/L, en particulier supérieure à 450 g/L.

La masse volumique tassée de ladite composition pulvérulente peut être mesurée à l'aide d'une éprouvette de 250 ml graduée tous les 2 ml ainsi qu'un voluminomètre type Dual Autotap conforme aux normes ASTM B527 et D4164. Le mode opératoire consiste à verser la composition pulvérulente dans l'éprouvette de manière à se situer proche de la graduation maximum du récipient. L'éprouvette est ensuite située délicatement sur le plateau de l'Autotap pour subir le nombre de secousses verticales désirées en fonction de la densité tassée recherchée (D10 = densité tassée à 10 coups, D500 = densité tassée à 500 coups). La masse volumique tassée est alors calculée par le rapport de la masse de poudre tassée sur le volume occupée par ladite poudre tassée dans ladite éprouvette.

Selon un mode de réalisation avantageux, la présente invention concerne une composition telle que définie précédemment, dont l'angle de talus est compris de 34° à 40°, en particulier de 36° à 38°.

L'angle de talus de ladite composition pulvérulente peut être mesurée en déterminant l'angle à la base du cône d'éboulement obtenu en faisant passer l'échantillon à travers un entonnoir spécial (entonnoir en acier inoxydable dont la base du diamètre (d) intérieur est de 6 mm) à une hauteur conventionnelle, au-dessus d'une plaque de marbre parfaitement plane et horizontale. Le mode opératoire, exécuté quatre fois, consiste à :

- ajuster la hauteur (H) entre la plaque et la base de l'entonnoir à 40 mm,
- vérifier l'aplomb de centre de l'entonnoir avec la plaque de marbre,
- disposer une feuille de papier sur la plaque en la centrant avec l'entonnoir,
- verser la poudre dans l'entonnoir,
- arrêter l'alimentation quand le sommet du cône touche la base de l'entonnoir,
- inscrire la base du cône, en forme de cercle, dans un carré,
- mesure la distance D séparant deux cotés opposés dudit carré.

L'angle de talus d'éboulement $\alpha$ est exprimé en degrés et est donné par la formule

$$\alpha = \mathrm{Arctg}\frac{2H}{D-d}$$

avec

H : la hauteur du cône en mm (H = 40),
d : diamètre intérieur de la base de l'entonnoir en mm (d = 6 mm),
D : moyenne arithmétique des 4 mesures en mm.

Selon un mode de réalisation avantageux, la présente invention concerne une composition telle que définie précédemment, comprenant, outre lesdites particules, de l'huile, notamment de l'huile végétale.

L'huile végétale est en particulier choisie parmi l'huile de soja, l'huile de tournesol, l'huile de colza, l'huile d'arachide et leurs mélanges.

La description concerne également un procédé de préparation d'une particule comprenant :

- un noyau constitué essentiellement d'un sel de formule (I) suivante :

$$(A^-)_n M^{n+} \qquad (I)$$

dans laquelle :

A⁻ représente un anion choisi dans le groupe constitué du 2-hydroxy-4-méthyl-thio-butanoate, du méthioniate et du cystéinate,
M représente un métal divalent ou trivalent,
n étant égal à 2 lorsque ledit métal est divalent et à 3 lorsque ledit métal est trivalent, et

- une couche comprenant un composé B choisi dans le groupe constitué de l'acide 2-hydroxy-4-méthyl-thio-butanoïque (HMTBA), de la méthionine, de la cystéine, de leurs mélanges, de leurs sels et de leurs complexes,

ladite couche enrobant ledit noyau,
le pourcentage massique dudit composé B par rapport au sel de formule (I) du noyau étant compris d'environ 10% à environ 50%,
ledit composé B n'étant pas, ou pas uniquement, sous la forme d'un sel de formule (I),
la teneur en composés organiques soufrés (TOS) de ladite particule étant supérieure à 87% en masse, en particulier supérieure à 88%, 89% ou 90% en masse,
ledit procédé comprenant une étape de pulvérisation sur un solide constitué essentiellement d'un sel de formule (I) telle que définie précédemment, d'une composition comprenant un composé B choisi dans le groupe constitué de l'acide 2-hydroxy-4-méthyl-thio-butanoïque (HMTBA), de la méthionine, de la cystéine, de leurs mélanges, de leurs sels et de leurs complexes, la masse dudit composé B étant comprise d'environ 10% à environ 50% de la masse du sel de formule (I) du solide,
pour obtenir ladite particule.

L'invention concerne également un procédé de préparation d'une particule comprenant :

- un noyau constitué essentiellement d'un sel de formule (I) suivante :

$$(A^-)_n M^{n+} \qquad (I)$$

dans laquelle :

A⁻ représente un anion choisi dans le groupe constitué du 2-hydroxy-4-méthyl-thio-butanoate, du méthioniate et du cystéinate,
M représente un métal divalent ou trivalent,
n étant égal à 2 lorsque ledit métal est divalent et à 3 lorsque ledit métal est trivalent. et

- une couche comprenant un composé B choisi dans le groupe constitué de l'acide 2-hydroxy-4-méthyl-thio-butanoïque (HMTBA).

ladite couche enrobant ledit noyau,
le pourcentage massique dudit composé B par rapport au sel de formule (I) du noyau étant compris de 10% à 50%.
ledit composé B n'étant pas, ou pas uniquement, sous la forme d'un sel de formule (I),
la teneur en composés organiques soufrés (TOS) de ladite particule étant supérieure à 87% en masse, en particulier supérieure à 88%, 89% ou 90% en masse, par rapport à la masse totale de ladite particule.
ledit procédé comprenant une étape de pulvérisation sur un solide constitué essentiellement d'un sel de formule (I) telle que définie précédemment, d'une composition comprenant un composé B choisi dans constitué de l'acide 2-hydroxy-4-méthyl-thio-butanoïque (HMTBA), la masse dudit composé B étant comprise de 10% à 50% de la masse du sel de formule (I) du solide,
pour obtenir ladite particule.

Selon un mode de réalisation avantageux, la présente description décrit un procédé tel que défini précédemment, dans lequel ladite composition comprend un composé B choisi dans le groupe constitué de l'acide 2-hydroxy-4-méthyl-thio-butanoïque (HMTBA), de la méthionine et de la cystéine, sous forme libre.

Selon un mode de réalisation avantageux, la présente invention concerne un procédé tel que défini précédemment, dans lequel ladite étape de pulvérisation est effectuée :

- en lit d'air fluidisé en discontinu ou en continu,
  ou
- en tour d'atomisation par copulvérisation.

Par copulvérisation, on entend la pulvérisation conjointe d'un liquide et d'une poudre.

Selon un mode de réalisation avantageux, la présente invention concerne un procédé tel que défini précédemment, dans lequel ladite étape de pulvérisation est réalisée en continu.

Selon un mode de réalisation avantageux, la présente invention concerne un procédé tel que défini précédemment, dans lequel ladite étape de pulvérisation est effectuée sur vibrofluidiseur.

Selon un mode de réalisation avantageux, la présente invention concerne un procédé tel que défini précédemment, dans lequel ladite étape de pulvérisation se fait sous atmosphère inerte, en particulier sous azote, le gaz inerte étant plus particulièrement recyclé.

Selon un mode de réalisation avantageux, la présente invention concerne un procédé tel que défini précédemment, dans lequel ladite composition est sous forme liquide.

Selon un mode de réalisation avantageux, la présente invention concerne un procédé tel que défini précédemment, dans lequel ladite composition comprend en outre de l'eau, le pourcentage massique en eau de ladite composition étant compris de 0,5 à 50.

Selon un mode de réalisation avantageux, la présente invention concerne un procédé tel que défini précédemment, dans lequel le noyau constitué essentiellement d'un sel de formule (I) est obtenu par atomisation réactive.

Par atomisation réactive, on entend l'atomisation d'un mélange réactionnel, ledit mélange étant la combinaison de 2 ou plusieurs composés qui peuvent réagir chimiquement ensemble lors de leur mélange, la mise en contact des composés du mélange réactionnel étant suivie immédiatement de la pulvérisation par atomisation.

Selon un mode de réalisation avantageux, la présente invention concerne un procédé tel que défini précédemment, dans lequel le noyau constitué essentiellement d'un sel de formule (I) est obtenu en lit d'air fluidisé, en granulateur, granulateur rotatif, ou en mélangeur.

Selon un mode de réalisation avantageux, la présente invention concerne un procédé tel que défini précédemment, dans lequel le noyau constitué essentiellement d'un sel de formule (I) est obtenu par extrusion réactive.

L'extrusion réactive peut être réalisée par des techniques bien connues de l'homme du métier. En particulier, le sel de formule (I) peut être obtenu par l'extrusion réactive telle que décrite dans la demande FR 2 964 968.

Selon un mode de réalisation avantageux, la présente invention concerne un procédé tel que défini précédemment, dans lequel le noyau constitué essentiellement d'un sel de formule (I) est obtenu à l'aide d'un mélangeur statique ou dynamique.

Selon un mode de réalisation avantageux, la présente invention concerne un procédé tel que défini précédemment, dans lequel le noyau constitué essentiellement d'un sel de formule (I) est obtenu par atomisation réactive sous atmosphère inerte, en particulier sous azote, le gaz inerte étant en particulier recyclé lorsque le noyau constitué essentiellement d'un sel de formule (I) n'est pas obtenu par extrusion réactive.

Selon un mode de réalisation avantageux, la présente description décrit un procédé tel que défini précédemment, dans lequel ledit composé B compris dans ladite couche est sous la forme :

- libre, choisie parmi l'acide 2-hydroxy-4-méthyl-thio-butanoïque (HMTBA), la méthionine et la cystéine, et/ou
- dudit sel de formule (I) tel que défini précédemment, et/ou
- d'un complexe de formule $(A)_4M$ (II) dans laquelle A et M sont tels que définis précédemment, A représentant de préférence l'acide 2-hydroxy-4-méthyl-thio-butanoïque (HMTBA),

ledit composé B n'étant pas, ou pas uniquement, sous la forme d'un sel de formule (I),
ledit composé B étant en particulier sous la forme :

- libre,
- du complexe de formule (II),
- d'un mélange de la forme libre et du complexe de formule (II),
- d'un mélange de la forme libre et du sel de formule (I),
- d'un mélange du sel de formule (I) et du complexe de formule (II), ou
- d'un mélange de la forme libre, du sel de formule (I) et du complexe de formule (II).

Selon un mode de réalisation avantageux, la présente invention concerne un procédé tel que défini précédemment, dans lequel ladite particule comprend moins de 3%, 2% ou 1,5%, d'eau en masse

Selon un mode de réalisation avantageux, la présente invention concerne un procédé tel que défini précédemment, dans lequel ladite particule a une teneur en calcium comprise de 6 à 11% en masse, en particulier de 6,5 à 10%, plus particulièrement de 7 à 9%, la teneur en calcium étant notamment d'environ 8%.

Selon un mode de réalisation avantageux, la présente invention concerne un procédé tel que défini précédemment, dans lequel le pourcentage massique dudit composé B par rapport au sel de formule (I) du noyau est compris d'environ 10% à environ 40%, en particulier d'environ 15 % à environ 35%, plus particulièrement d'environ 20% à environ 32%.

Selon un mode de réalisation avantageux, la présente invention concerne un procédé tel que défini précédemment, dans lequel ledit métal est choisi dans le groupe comprenant Mg, Be, Ca, Sr, Ba, Mn, Fe, Co, Ni, Cu, Zn, Pt, B, Al, Ga, In, en particulier Mg, Ca, Fe, Mn, Al, Cu, Zn.

Selon un mode de réalisation avantageux, la présente invention concerne un procédé tel que défini précédemment, dans lequel ledit métal et n sont tels que $M^{n+}$ représente $Mg^{2+}$, $Be^{2+}$, $Ca^{2+}$, $Sr^{2+}$, $Ba^{2+}$, $Mn^{2+}$, $Fe^{2+}$, $Fe^{3+}$, $Co^{2+}$, $Co^{3+}$, $Ni^{2+}$, $Ni^{3+}$, $Cu^{2+}$, $Zn^{2+}$, $Pt^{2+}$, $Al^{3+}$, $Ga^{3+}$ ou $In^{3+}$, en particulier $Mg^{2+}$, $Ca^{2+}$, $Fe^{2+}$, $Fe^{3+}$, $Al^{3+}$, $Mn^{2+}$, $Cu^{2+}$ ou $Zn^{2+}$.

Selon un mode de réalisation avantageux, la présente invention concerne un procédé tel que défini précédemment, dans lequel ledit sel de formule (I) est (HMTBA)$_2$Ca, (HMTBA)$_2$Mg, (HMTBA)$_2$Fe, (HMTBA)$_2$Mn, (HMTBA)$_2$Zn, (HMTBA)$_2$Cu, (HMTBA)$_3$Fe, (HMTBA)$_3$Al, (Méthionine)$_2$Ca, (Méthionine)$_2$Mg, (Méthionine)$_2$Fe, (Méthionine)$_2$Mn, (Méthionine)$_2$Zn, (Méthionine)$_2$Cu, (Méthionine)$_3$Fe, (Méthionine)$_3$Al, (Cystéine)$_2$Ca, (Cystéine)$_2$Mg, (Cystéine)$_2$Fe, (Cystéine)$_2$Mn, (Cystéine)$_2$Zn, (Cystéine)$_2$Cu, (Cystéine)$_3$Fe, ou (Cystéine)$_3$Al, encore plus particulièrement un sel de formule (HMTBA)$_2$Ca, (HMTBA)$_2$Mg, (HMTBA)$_2$Fe, (HMTBA)$_2$Mn, (HMTBA)$_2$Zn, (HMTBA)$_2$Cu, (Méthionine)$_2$Ca, (Méthionine)$_2$Mg, (Méthionine)$_2$Fe, (Méthionine)$_2$Mn, (Méthionine)$_2$Zn, (Méthionine)$_2$Cu, (Cystéine)$_2$Ca, (Cystéine)$_2$Mg, (Cystéine)$_2$Fe, (Cystéine)$_2$Mn, (Cystéine)$_2$Zn ou (Cystéine)$_2$Cu.

Selon un mode de réalisation avantageux, la présente invention concerne un procédé tel que défini précédemment, dans lequel ledit anion $A^-$ est le 2-hydroxy-4-méthyl-thio-butanoate.

Selon un mode de réalisation avantageux, la présente invention concerne un procédé tel que défini précédemment, dans lequel ledit composé B compris dans ladite couche est l'acide 2-hydroxy-4-méthyl-thio-butanoïque (HMTBA), ou l'un de ses sels ou complexes.

Selon un mode de réalisation avantageux, la présente invention concerne un procédé tel que défini précédemment, dans lequel ledit anion $A^-$ est le 2-hydroxy-4-méthyl-thio-butanoate, et ledit composé B compris dans ladite couche est l'acide 2-hydroxy-4-méthyl-thio-butanoïque (HMTBA), ou l'un de ses sels ou complexes.

Selon un mode de réalisation avantageux, la présente invention concerne un procédé tel que défini précédemment, dans lequel ledit acide 2-hydroxy-4-méthyl-thio-butanoïque (HMTBA) est sous forme monomérique à plus de 60% en masse.

Selon un mode de réalisation avantageux, la présente description décrit un procédé de préparation d'une particule comprenant :

- un noyau constitué essentiellement de sel de formule (I) (HMTBA)$_2$Ca, et
- une couche comprenant un composé B choisi dans le groupe constitué de l'acide 2-hydroxy-4-méthyl-thio-butanoïque (HMTBA), de la méthionine, de la cystéine, de leurs mélanges, de leurs sels et de leurs complexes,

ladite couche enrobant ledit noyau,
le pourcentage massique dudit composé B par rapport au sel de formule (I) (HMTBA)$_2$Ca du noyau étant compris d'environ 10% à environ 50%,
ledit composé B n'étant pas, ou pas uniquement, sous la forme du sel de formule (I) (HMTBA)$_2$Ca,
la teneur en composés organiques soufrés (TOS) de ladite particule étant supérieure à 87% en masse, en particulier supérieure à 88%, 89% ou 90% en masse, par rapport à la masse totale de ladite particule,
ledit procédé comprenant une étape de pulvérisation sur un solide constitué essentiellement de sel de formule (I) (HMTBA)$_2$Ca, d'une composition comprenant un composé B choisi dans le groupe constitué de l'acide 2-hydroxy-4-méthyl-thio-butanoïque (HMTBA), de la méthionine, de la cystéine, de leurs mélanges, de leurs sels et de leurs complexes, la masse dudit composé B étant comprise d'environ 10% à environ 50% de la masse du sel de formule (I) du solide, pour obtenir ladite particule.

Selon un mode de réalisation avantageux, la présente description décrit un procédé de préparation d'une particule comprenant :

- un noyau constitué essentiellement de sel de formule (I) (HMTBA)$_2$Ca, et
- une couche comprenant un composé B choisi dans le groupe constitué de l'acide 2-hydroxy-4-méthyl-thio-butanoïque (HMTBA), de la méthionine, de la cystéine, de leurs mélanges, de leurs sels et de leurs complexes,

ladite couche enrobant ledit noyau,

le pourcentage massique dudit composé B par rapport au sel de formule (I) (HMTBA)$_2$Ca du noyau étant compris d'environ 10% à environ 50%,

ledit composé B n'étant pas, ou pas uniquement, sous la forme du sel de formule (I) (HMTBA)$_2$Ca,

la teneur en composés organiques soufrés (TOS) de ladite particule étant supérieure à 87% en masse, en particulier supérieure à 88%, 89% ou 90% en masse, par rapport à la masse totale de ladite particule,

ledit procédé comprenant une étape de pulvérisation en lit d'air fluidisé en discontinu ou en continu sur un solide constitué essentiellement de sel de formule (I) (HMTBA)$_2$Ca, d'une composition comprenant un composé B choisi dans le groupe constitué de l'acide 2-hydroxy-4-méthyl-thio-butanoïque (HMTBA), de la méthionine, de la cystéine, de leurs mélanges, de leurs sels et de leurs complexes, la masse dudit composé B étant comprise d'environ 10% à environ 50% de la masse du sel de formule (I) du solide,

pour obtenir ladite particule.

Selon un mode de réalisation avantageux, la présente description décrit un procédé de préparation d'une particule comprenant :

- un noyau constitué essentiellement de sel de formule (I) (HMTBA)$_2$Ca, et
- une couche comprenant un composé B choisi dans le groupe constitué de l'acide 2-hydroxy-4-méthyl-thio-butanoïque (HMTBA), de la méthionine, de la cystéine, de leurs mélanges, de leurs sels et de leurs complexes,

ladite couche enrobant ledit noyau,

le pourcentage massique dudit composé B par rapport au sel de formule (I) (HMTBA)$_2$Ca du noyau étant compris d'environ 10% à environ 50%,

ledit composé B n'étant pas, ou pas uniquement, sous la forme du sel de formule (I) (HMTBA)$_2$Ca,

la teneur en composés organiques soufrés (TOS) de ladite particule étant supérieure à 87% en masse, en particulier supérieure à 88%, 89% ou 90% en masse, par rapport à la masse totale de ladite particule,

ledit procédé comprenant une étape de pulvérisation en lit d'air fluidisé en discontinu ou en continu sur un solide constitué essentiellement de sel de formule (I) (HMTBA)$_2$Ca, ledit solide étant obtenu par atomisation réactive, d'une composition comprenant un composé B choisi dans le groupe constitué de l'acide 2-hydroxy-4-méthyl-thio-butanoïque (HMTBA), de la méthionine, de la cystéine, de leurs mélanges, de leurs sels et de leurs complexes, la masse dudit composé B étant comprise d'environ 10% à environ 50% de la masse du sel de formule (I) du solide,

pour obtenir ladite particule.

Selon un mode de réalisation avantageux, la présente description décrit un procédé de préparation d'une particule comprenant :

- un noyau constitué essentiellement de sel de formule (I) (HMTBA)$_2$Ca, et
- une couche comprenant un composé B choisi dans le groupe constitué de l'acide 2-hydroxy-4-méthyl-thio-butanoïque (HMTBA), de la méthionine, de la cystéine, de leurs mélanges, de leurs sels et de leurs complexes,

ladite couche enrobant ledit noyau,

le pourcentage massique dudit composé B par rapport au sel de formule (I) (HMTBA)$_2$Ca du noyau étant compris d'environ 10% à environ 50%,

ledit composé B n'étant pas, ou pas uniquement, sous la forme du sel de formule (I) (HMTBA)$_2$Ca,

la teneur en composés organiques soufrés (TOS) de ladite particule étant supérieure à 87% en masse, en particulier supérieure à 88%, 89% ou 90% en masse, par rapport à la masse totale de ladite particule,

ledit procédé comprenant une étape de pulvérisation en tour d'atomisation par copulvérisation sur un solide constitué essentiellement de sel de formule (I) (HMTBA)$_2$Ca, d'une composition comprenant un composé B choisi dans le groupe constitué de l'acide 2-hydroxy-4-méthyl-thio-butanoïque (HMTBA), de la méthionine, de la cystéine, de leurs mélanges, de leurs sels et de leurs complexes, la masse dudit composé B étant comprise d'environ 10% à environ 50% de la masse du sel de formule (I) du solide,

pour obtenir ladite particule.

Selon un mode de réalisation avantageux, la présente description décrit un procédé de préparation d'une particule comprenant :

- un noyau constitué essentiellement de sel de formule (I) (HMTBA)$_2$Ca, et
- une couche comprenant un composé B choisi dans le groupe constitué de l'acide 2-hydroxy-4-méthyl-thio-butanoïque (HMTBA), de la méthionine, de la cystéine, de leurs mélanges, de leurs sels et de leurs complexes,

ladite couche enrobant ledit noyau,

le pourcentage massique dudit composé B par rapport au sel de formule (I) (HMTBA)$_2$Ca du noyau étant compris d'environ 10% à environ 50%,

ledit composé B n'étant pas, ou pas uniquement, sous la forme du sel de formule (I) (HMTBA)$_2$Ca,

la teneur en composés organiques soufrés (TOS) de ladite particule étant supérieure à 87% en masse, en particulier supérieure à 88%, 89% ou 90% en masse, par rapport à la masse totale de ladite particule,

ledit procédé comprenant une étape de pulvérisation en tour d'atomisation par copulvérisation sur un solide constitué essentiellement de sel de formule (I) (HMTBA)$_2$Ca, ledit solide étant obtenu par atomisation réactive dans ladite tour d'atomisation, d'une composition comprenant un composé B choisi dans le groupe constitué de l'acide 2-hydroxy-4-méthyl-thio-butanoïque (HMTBA), de la méthionine, de la cystéine, de leurs mélanges, de leurs sels et de leurs complexes, la masse dudit composé B étant comprise d'environ 10% à environ 50% de la masse du sel de formule (I) du solide, pour obtenir ladite particule.

Selon un mode de réalisation avantageux, la présente invention concerne un procédé de préparation d'une particule comprenant :

- un noyau constitué essentiellement de sel de formule (I) (HMTBA)$_2$Ca, et
- une couche comprenant de l'acide 2-hydroxy-4-méthyl-thio-butanoïque (HMTBA),

ladite couche enrobant ledit noyau,

le pourcentage massique du HMTBA de ladite couche par rapport au sel de formule (I) (HMTBA)$_2$Ca du noyau étant compris d'environ 10% à environ 50%,

le HMTBA de ladite couche n'étant pas, ou pas uniquement, sous la forme du sel de formule (I) (HMTBA)$_2$Ca,

la teneur en composés organiques soufrés (TOS) de ladite particule étant supérieure à 87% en masse, en particulier supérieure à 88%, 89% ou 90% en masse, par rapport à la masse totale de ladite particule,

ledit procédé comprenant une étape de pulvérisation sur un solide constitué essentiellement de sel de formule (I) (HMTBA)$_2$Ca, d'une composition comprenant de l'acide 2-hydroxy-4-méthyl-thio-butanoïque (HMTBA), la masse dudit composé B étant comprise d'environ 10% à environ 50% de la masse du sel de formule (I) du solide, pour obtenir ladite particule.

Selon un mode de réalisation avantageux, la présente invention concerne un procédé de préparation d'une particule comprenant :

- un noyau constitué essentiellement de sel de formule (I) (HMTBA)$_2$Ca, et
- une couche comprenant de l'acide 2-hydroxy-4-méthyl-thio-butanoïque (HMTBA),

ladite couche enrobant ledit noyau,

le pourcentage massique du HMTBA de ladite couche par rapport au sel de formule (I) (HMTBA)$_2$Ca du noyau étant compris d'environ 10% à environ 50%,

le HMTBA de ladite couche n'étant pas, ou pas uniquement, sous la forme du sel de formule (I) (HMTBA)$_2$Ca,

la teneur en composés organiques soufrés (TOS) de ladite particule étant supérieure à 87% en masse, en particulier supérieure à 88%, 89% ou 90% en masse, par rapport à la masse totale de ladite particule,

ledit procédé comprenant une étape de pulvérisation en lit d'air fluidisé en discontinu ou en continu sur un solide constitué essentiellement de sel de formule (I) (HMTBA)$_2$Ca, d'une composition comprenant de l'acide 2-hydroxy-4-méthyl-thio-butanoïque (HMTBA), la masse dudit composé B étant comprise d'environ 10% à environ 50% de la masse du sel de formule (I) du solide, pour obtenir ladite particule.

Selon un mode de réalisation avantageux, la présente invention concerne un procédé de préparation d'une particule comprenant :

- un noyau constitué essentiellement de sel de formule (I) (HMTBA)$_2$Ca, et
- une couche comprenant de l'acide 2-hydroxy-4-méthyl-thio-butanoïque (HMTBA), ou l'un de ses sels ou complexes,

ladite couche enrobant ledit noyau,

le pourcentage massique du HMTBA de ladite couche par rapport au sel de formule (I) (HMTBA)$_2$Ca du noyau étant compris d'environ 10% à environ 50%,

le HMTBA de ladite couche n'étant pas, ou pas uniquement, sous la forme du sel de formule (I) (HMTBA)$_2$Ca,

la teneur en composés organiques soufrés (TOS) de ladite particule étant supérieure à 87% en masse, en particulier supérieure à 88%, 89% ou 90% en masse, par rapport à la masse totale de ladite particule,

ledit procédé comprenant une étape de pulvérisation en lit d'air fluidisé en discontinu ou en continu sur un solide constitué essentiellement de sel de formule (I) (HMTBA)$_2$Ca, ledit solide étant obtenu par atomisation réactive, d'une composition comprenant de l'acide 2-hydroxy-4-méthyl-thio-butanoïque (HMTBA), la masse dudit HMTBA de ladite couche étant comprise d'environ 10% à environ 50% de la masse du sel de formule (I) du solide, pour obtenir ladite particule.

Selon un mode de réalisation avantageux, la présente invention concerne un procédé de préparation d'une particule comprenant :

- un noyau constitué essentiellement de sel de formule (I) (HMTBA)$_2$Ca, et
- une couche comprenant de l'acide 2-hydroxy-4-méthyl-thio-butanoïque (HMTBA), ou l'un de ses sels ou complexes,

ladite couche enrobant ledit noyau,
le pourcentage massique du HMTBA de ladite couche par rapport au sel de formule (I) (HMTBA)$_2$Ca du noyau étant compris d'environ 10% à environ 50%,
le HMTBA de ladite couche n'étant pas, ou pas uniquement, sous la forme du sel de formule (I) (HMTBA)$_2$Ca,
la teneur en composés organiques soufrés (TOS) de ladite particule étant supérieure à 87% en masse, en particulier supérieure à 88%, 89% ou 90% en masse, par rapport à la masse totale de ladite particule,
ledit procédé comprenant une étape de pulvérisation en tour d'atomisation par copulvérisation sur un solide constitué essentiellement de sel de formule (I) (HMTBA)$_2$Ca, d'une composition comprenant de l'acide 2-hydroxy-4-méthyl-thio-butanoïque (HMTBA), la masse dudit HMTBA de ladite couche étant comprise d'environ 10% à environ 50% de la masse du sel de formule (I) du solide,
pour obtenir ladite particule.

Selon un mode de réalisation avantageux, la présente invention concerne un procédé de préparation d'une particule comprenant :

- un noyau constitué essentiellement de sel de formule (I) (HMTBA)$_2$Ca, et
- une couche comprenant de l'acide 2-hydroxy-4-méthyl-thio-butanoïque (HMTBA), ou l'un de ses sels ou complexes,

ladite couche enrobant ledit noyau,
le pourcentage massique du HMTBA de ladite couche par rapport au sel de formule (I) (HMTBA)$_2$Ca du noyau étant compris d'environ 10% à environ 50%,
le HMTBA de ladite couche n'étant pas, ou pas uniquement, sous la forme du sel de formule (I) (HMTBA)$_2$Ca,
la teneur en composés organiques soufrés (TOS) de ladite particule étant supérieure à 87% en masse, en particulier supérieure à 88%, 89% ou 90% en masse, par rapport à la masse totale de ladite particule,
ledit procédé comprenant une étape de pulvérisation en tour d'atomisation par copulvérisation sur un solide constitué essentiellement de sel de formule (I) (HMTBA)$_2$Ca, ledit solide étant obtenu par atomisation réactive dans ladite tour d'atomisation, d'une composition comprenant de l'acide 2-hydroxy-4-méthyl-thio-butanoïque (HMTBA), la masse dudit HMTBA de ladite couche étant comprise d'environ 10% à environ 50% de la masse du sel de formule (I) du solide, pour obtenir ladite particule.

Selon un mode de réalisation avantageux, la présente invention concerne un procédé de préparation d'une particule comprenant :

- un noyau constitué essentiellement de sel de formule (I) (HMTBA)$_2$Ca, et
- une couche comprenant de l'acide 2-hydroxy-4-méthyl-thio-butanoïque (HMTBA) ou l'un de ses sels ou complexes, et du calcium, le rapport atomique S/Ca étant compris de 2,7 à 3,7,

ladite couche enrobant ledit noyau,
le pourcentage massique du HMTBA de ladite couche par rapport au sel de formule (I) du noyau étant compris d'environ 10% à environ 50%,
la teneur en composés organiques soufrés (TOS) de ladite particule étant supérieure à 87% en masse, en particulier supérieure à 88%, 89% ou 90% en masse, par rapport à la masse totale de ladite particule.,
ledit procédé comprenant une étape de pulvérisation sur un solide constitué essentiellement de sel de formule (I) (HMTBA)$_2$Ca, d'une composition comprenant de l'acide 2-hydroxy-4-méthyl-thio-butanoïque (HMTBA), la masse dudit HMTBA de ladite couche étant comprise d'environ 10% à environ 50% de la masse du sel de formule (I) du solide, pour obtenir ladite particule.

Selon un mode de réalisation avantageux, la présente invention concerne un procédé de préparation d'une particule comprenant :

- un noyau constitué essentiellement de sel de formule (I) (HMTBA)$_2$Ca, et
- une couche comprenant de l'acide 2-hydroxy-4-méthyl-thio-butanoïque (HMTBA) ou l'un de ses sels ou complexes, et du calcium, le rapport atomique S/Ca étant compris de 2,7 à 3,7,

ladite couche enrobant ledit noyau,
le pourcentage massique du HMTBA de ladite couche par rapport au sel de formule (I) du noyau étant compris d'environ 10% à environ 50%,
la teneur en composés organiques soufrés (TOS) de ladite particule étant supérieure à 87% en masse, en particulier supérieure à 88%, 89% ou 90% en masse, par rapport à la masse totale de ladite particule.,
ledit procédé comprenant une étape de pulvérisation en lit d'air fluidisé en discontinu ou en continu sur un solide constitué essentiellement de sel de formule (I) (HMTBA)$_2$Ca, d'une composition comprenant de l'acide 2-hydroxy-4-méthyl-thio-butanoïque (HMTBA), la masse dudit HMTBA de ladite couche étant comprise d'environ 10% à environ 50% de la masse du sel de formule (I) du solide,
pour obtenir ladite particule.

Selon un mode de réalisation avantageux, la présente invention concerne un procédé de préparation d'une particule comprenant :

- un noyau constitué essentiellement de sel de formule (I) (HMTBA)$_2$Ca, et
- une couche comprenant de l'acide 2-hydroxy-4-méthyl-thio-butanoïque (HMTBA) ou l'un de ses sels ou complexes, et du calcium, le rapport atomique S/Ca étant compris de 2,7 à 3,7,

ladite couche enrobant ledit noyau,
le pourcentage massique du HMTBA de ladite couche par rapport au sel de formule (I) du noyau étant compris d'environ 10% à environ 50%,
la teneur en composés organiques soufrés (TOS) de ladite particule étant supérieure à 87% en masse, en particulier supérieure à 88%, 89% ou 90% en masse, par rapport à la masse totale de ladite particule.,
ledit procédé comprenant une étape de pulvérisation en lit d'air fluidisé en discontinu ou en continu sur un solide constitué essentiellement de sel de formule (I) (HMTBA)$_2$Ca, ledit solide étant obtenu par atomisation réactive, d'une composition comprenant de l'acide 2-hydroxy-4-méthyl-thio-butanoïque (HMTBA), la masse dudit HMTBA de ladite couche étant comprise d'environ 10% à environ 50% de la masse du sel de formule (I) du solide,
pour obtenir ladite particule.

Selon un mode de réalisation avantageux, la présente invention concerne un procédé de préparation d'une particule comprenant :

- un noyau constitué essentiellement de sel de formule (I) (HMTBA)$_2$Ca, et
- une couche comprenant de l'acide 2-hydroxy-4-méthyl-thio-butanoïque (HMTBA) ou l'un de ses sels ou complexes, et du calcium, le rapport atomique S/Ca étant compris de 2,7 à 3,7,

ladite couche enrobant ledit noyau,
le pourcentage massique du HMTBA de ladite couche par rapport au sel de formule (I) du noyau étant compris d'environ 10% à environ 50%,
la teneur en composés organiques soufrés (TOS) de ladite particule étant supérieure à 87% en masse, en particulier supérieure à 88%, 89% ou 90% en masse, par rapport à la masse totale de ladite particule.,
ledit procédé comprenant une étape de pulvérisation en tour d'atomisation par copulvérisation sur un solide constitué essentiellement de sel de formule (I) (HMTBA)$_2$Ca, d'une composition comprenant de l'acide 2-hydroxy-4-méthyl-thio-butanoïque (HMTBA), la masse dudit HMTBA de ladite couche étant comprise d'environ 10% à environ 50% de la masse du sel de formule (I) du solide,
pour obtenir ladite particule.

Selon un mode de réalisation avantageux, la présente invention concerne un procédé de préparation d'une particule comprenant :

- un noyau constitué essentiellement de sel de formule (I) (HMTBA)$_2$Ca, et
- une couche comprenant de l'acide 2-hydroxy-4-méthyl-thio-butanoïque (HMTBA) ou l'un de ses sels ou complexes, et du calcium, le rapport atomique S/Ca étant compris de 2,7 à 3,7,

ladite couche enrobant ledit noyau,
le pourcentage massique du HMTBA de ladite couche par rapport au sel de formule (I) du noyau étant compris d'environ 10% à environ 50%,
la teneur en composés organiques soufrés (TOS) de ladite particule étant supérieure à 87% en masse, en particulier supérieure à 88%, 89% ou 90% en masse, par rapport à la masse totale de ladite particule.,

ledit procédé comprenant une étape de pulvérisation en tour d'atomisation par copulvérisation sur un solide constitué essentiellement de sel de formule (I) $(HMTBA)_2Ca$, ledit solide étant obtenu par atomisation réactive dans ladite tour d'atomisation, d'une composition comprenant de l'acide 2-hydroxy-4-méthyl-thio-butanoïque (HMTBA), la masse dudit HMTBA de ladite couche étant comprise d'environ 10% à environ 50% de la masse du sel de formule (I) du solide, pour obtenir ladite particule.

Selon un mode de réalisation avantageux, la présente invention concerne un procédé de préparation d'une particule comprenant :

- un noyau constitué essentiellement de sel de formule (I) $(HMTBA)_2Ca$, et
- une couche comprenant du complexe de formule (II) $(HMTBA)_4Ca$,

ladite couche enrobant ledit noyau,
le pourcentage massique du HMTBA de ladite couche par rapport au sel de formule (I) du noyau étant compris d'environ 10% à environ 50%,
la teneur en composés organiques soufrés (TOS) de ladite particule étant supérieure à 87% en masse, en particulier supérieure à 88%, 89% ou 90% en masse, par rapport à la masse totale de ladite particule.,
ledit procédé comprenant une étape de pulvérisation sur un solide constitué essentiellement de sel de formule (I) $(HMTBA)_2Ca$, d'une composition comprenant de l'acide 2-hydroxy-4-méthyl-thio-butanoïque (HMTBA), la masse dudit HMTBA de ladite couche étant comprise d'environ 10% à environ 50% de la masse du sel de formule (I) du solide, pour obtenir ladite particule.

Selon un mode de réalisation avantageux, la présente invention concerne un procédé de préparation d'une particule comprenant :

- un noyau constitué essentiellement de sel de formule (I) $(HMTBA)_2Ca$, et
- une couche comprenant du complexe de formule (II) $(HMTBA)_4Ca$,

ladite couche enrobant ledit noyau,
le pourcentage massique du HMTBA de ladite couche par rapport au sel de formule (I) du noyau étant compris d'environ 10% à environ 50%,
la teneur en composés organiques soufrés (TOS) de ladite particule étant supérieure à 87% en masse, en particulier supérieure à 88%, 89% ou 90% en masse, par rapport à la masse totale de ladite particule.,
ledit procédé comprenant une étape de pulvérisation en lit d'air fluidisé en discontinu ou en continu sur un solide constitué essentiellement de sel de formule (I) $(HMTBA)_2Ca$, d'une composition comprenant de l'acide 2-hydroxy-4-méthyl-thio-butanoïque (HMTBA), la masse dudit HMTBA de ladite couche étant comprise d'environ 10% à environ 50% de la masse du sel de formule (I) du solide, pour obtenir ladite particule.

Selon un mode de réalisation avantageux, la présente invention concerne un procédé de préparation d'une particule comprenant :

- un noyau constitué essentiellement de sel de formule (I) $(HMTBA)_2Ca$, et
- une couche comprenant du complexe de formule (II) $(HMTBA)_4Ca$,

ladite couche enrobant ledit noyau,
le pourcentage massique du HMTBA de ladite couche par rapport au sel de formule (I) du noyau étant compris d'environ 10% à environ 50%,
la teneur en composés organiques soufrés (TOS) de ladite particule étant supérieure à 87% en masse, en particulier supérieure à 88%, 89% ou 90% en masse, par rapport à la masse totale de ladite particule.,
ledit procédé comprenant une étape de pulvérisation en lit d'air fluidisé en discontinu ou en continu sur un solide constitué essentiellement de sel de formule (I) $(HMTBA)_2Ca$, ledit solide étant obtenu par atomisation réactive, d'une composition comprenant de l'acide 2-hydroxy-4-méthyl-thio-butanoïque (HMTBA), la masse dudit HMTBA de ladite couche étant comprise d'environ 10% à environ 50% de la masse du sel de formule (I) du solide, pour obtenir ladite particule.

Selon un mode de réalisation avantageux, la présente invention concerne un procédé de préparation d'une particule comprenant :

- un noyau constitué essentiellement de sel de formule (I) $(HMTBA)_2Ca$, et
- une couche comprenant du complexe de formule (II) $(HMTBA)_4Ca$,

ladite couche enrobant ledit noyau,
le pourcentage massique du HMTBA de ladite couche par rapport au sel de formule (I) du noyau étant compris d'environ 10% à environ 50%,
la teneur en composés organiques soufrés (TOS) de ladite particule étant supérieure à 87% en masse, en particulier supérieure à 88%, 89% ou 90% en masse, par rapport à la masse totale de ladite particule.,
ledit procédé comprenant une étape de pulvérisation en tour d'atomisation par copulvérisation sur un solide constitué essentiellement de sel de formule (I) $(HMTBA)_2Ca$, d'une composition comprenant de l'acide 2-hydroxy-4-méthyl-thio-butanoïque (HMTBA), la masse dudit HMTBA de ladite couche étant comprise d'environ 10% à environ 50% de la masse du sel de formule (I) du solide,
pour obtenir ladite particule.

Selon un mode de réalisation avantageux, la présente invention concerne un procédé de préparation d'une particule comprenant :

- un noyau constitué essentiellement de sel de formule (I) $(HMTBA)_2Ca$, et
- une couche comprenant du complexe de formule (II) $(HMTBA)_4Ca$,

ladite couche enrobant ledit noyau,
le pourcentage massique du HMTBA de ladite couche par rapport au sel de formule (I) du noyau étant compris d'environ 10% à environ 50%,
la teneur en composés organiques soufrés (TOS) de ladite particule étant supérieure à 87% en masse, en particulier supérieure à 88%, 89% ou 90% en masse, par rapport à la masse totale de ladite particule.,
ledit procédé comprenant une étape de pulvérisation en tour d'atomisation par copulvérisation sur un solide constitué essentiellement de sel de formule (I) $(HMTBA)_2Ca$, ledit solide étant obtenu par atomisation réactive dans ladite tour d'atomisation, d'une composition comprenant de l'acide 2-hydroxy-4-méthyl-thio-butanoïque (HMTBA), la masse dudit HMTBA de ladite couche étant comprise d'environ 10% à environ 50% de la masse du sel de formule (I) du solide,
pour obtenir ladite particule.

Selon un mode de réalisation avantageux, la présente invention concerne un procédé de préparation d'une particule dans laquelle le composé A formant ledit anion $A^-$ et ledit composé B si B est sous forme libre, ou le composé formant le composé B, si B est sous forme de sel ou de complexe, sont différents.

Selon un mode de réalisation avantageux, la présente description décrit un procédé de préparation d'une particule dans laquelle le composé A formant ledit anion $A^-$ et ledit composé B si B est sous forme libre, ou le composé formant le composé B, si B est sous forme de sel ou de complexe, sont différents,
ledit procédé comprenant une étape de pulvérisation sur un solide constitué essentiellement d'un sel de formule (I) $(A^-)_nM^{n+}$ tel que défini précédement, d'une composition comprenant un composé B choisi dans le groupe constitué de l'acide 2-hydroxy-4-méthyl-thio-butanoïque (HMTBA), de la méthionine et de la cystéine, de leurs mélanges, de leurs sels et de leurs complexes, la masse dudit composé B étant comprise d'environ 10% à environ 50% de la masse du sel de formule (I) du solide,
pour obtenir ladite particule.

Selon un mode de réalisation avantageux, la présente invention concerne un procédé de préparation d'une particule dans laquelle le composé A formant ledit anion $A^-$ et ledit composé B si B est sous forme libre, ou le composé formant le composé B, si B est sous forme de sel ou de complexe, sont différents.

Selon un mode de réalisation avantageux, la présente description décrit un procédé de préparation d'une particule dans laquelle le composé A formant ledit anion $A^-$ et ledit composé B si B est sous forme libre, ou le composé formant le composé B, si B est sous forme de sel ou de complexe, sont différents,
ledit procédé comprenant une étape de pulvérisation en lit d'air fluidisé en discontinu ou en continu sur un solide constitué essentiellement d'un sel de formule (I) $(A^-)_nM^{n+}$ tel que défini précédement, d'une composition comprenant un composé B choisi dans le groupe constitué de l'acide 2-hydroxy-4-méthyl-thio-butanoïque (HMTBA), de la méthionine et de la cystéine, de leurs mélanges, de leurs sels et de leurs complexes, la masse dudit composé B étant comprise d'environ 10% à environ 50% de la masse du sel de formule (I) du solide,
pour obtenir ladite particule.

Selon un mode de réalisation avantageux, la présente invention concerne un procédé de préparation d'une particule dans laquelle le composé A formant ledit anion $A^-$ et ledit composé B si B est sous forme libre, ou le composé formant le composé B, si B est sous forme de sel ou de complexe, sont différents.

Selon un mode de réalisation avantageux, la présente description décrit un procédé de préparation d'une particule dans laquelle le composé A formant ledit anion $A^-$ et ledit composé B si B est sous forme libre, ou le composé formant le composé B, si B est sous forme de sel ou de complexe, sont différents,
ledit procédé comprenant une étape de pulvérisation en lit d'air fluidisé en discontinu ou en continu sur un solide constitué essentiellement d'un sel de formule (I) $(A^-)_nM^{n+}$ tel que défini précédement, ledit solide étant obtenu par atomisation réactive, d'une composition comprenant un composé B choisi dans le groupe constitué de l'acide 2-hydroxy-4-méthyl-thio-butanoïque (HMTBA), de la méthionine et de la cystéine, de leurs mélanges, de leurs sels et de leurs complexes, la masse dudit composé B étant comprise d'environ 10% à environ 50% de la masse du sel de formule (I) du solide, pour obtenir ladite particule.

Selon un mode de réalisation avantageux, la présente description décrit un procédé de préparation d'une particule dans laquelle le composé A formant ledit anion A⁻ et ledit composé B si B est sous forme libre, ou le composé formant le composé B, si B est sous forme de sel ou de complexe, sont différents.

Selon un mode de réalisation avantageux, la présente invention concerne un procédé de préparation d'une particule dans laquelle le composé A formant ledit anion A⁻ et ledit composé B si B est sous forme libre, ou le composé formant le composé B, si B est sous forme de sel ou de complexe, sont différents, ledit procédé comprenant une étape de pulvérisation en tour d'atomisation par copulvérisation sur un solide constitué essentiellement d'un sel de formule (I) $(A^-)_n M^{n+}$ tel que défini précédemment, d'une composition comprenant un composé B choisi dans le groupe constitué de l'acide 2-hydroxy-4-méthyl-thio-butanoïque (HMTBA), de la méthionine et de la cystéine, de leurs mélanges, de leurs sels et de leurs complexes, la masse dudit composé B étant comprise d'environ 10% à environ 50% de la masse du sel de formule (I) du solide, pour obtenir ladite particule.

Selon un mode de réalisation avantageux, la présente invention concerne un procédé de préparation d'une particule dans laquelle le composé A formant ledit anion A⁻ et ledit composé B si B est sous forme libre, ou le composé formant le composé B, si B est sous forme de sel ou de complexe, sont différents.

Selon un mode de réalisation avantageux, la présente description décrit un procédé de préparation d'une particule dans laquelle le composé A formant ledit anion A⁻ et ledit composé B si B est sous forme libre, ou le composé formant le composé B, si B est sous forme de sel ou de complexe, sont différents, ledit procédé comprenant une étape de pulvérisation en tour d'atomisation par copulvérisation sur un solide constitué essentiellement d'un sel de formule (I) $(A^-)_n M^{n+}$ tel que défini précédemment, ledit solide étant obtenu par atomisation réactive dans ladite tour d'atomisation, d'une composition comprenant un composé B choisi dans le groupe constitué de l'acide 2-hydroxy-4-méthyl-thio-butanoïque (HMTBA), de la méthionine et de la cystéine, de leurs mélanges, de leurs sels et de leurs complexes, la masse dudit composé B étant comprise d'environ 10% à environ 50% de la masse du sel de formule (I) du solide, pour obtenir ladite particule.

## DESCRIPTION DES FIGURES

La **figure 1A** est un cliché de microscopie optique relatif à la poudre obtenue à l'issue de l'exemple 1.

La **figure 1B** est un cliché de microscopie optique relatif à la poudre obtenue à l'issue de l'exemple 12.

La **figure 2A** est un cliché de microscopie optique à balayage relatif à la poudre obtenue à l'issue de l'exemple 1.

La **figure 2B** est un cliché de microscopie optique à balayage relatif à la poudre obtenue à l'issue de l'exemple 12.

La **figure 3A** présente le spectre d'analyse aux rayons X de poudres de l'échantillon A entre $2\theta=1°$ et $2\theta=24°$ obtenues à l'aide d'une radiation Mo-K$\alpha$ ($\lambda$ = 0,71073Å).

La **figure 3B** présente le spectre d'analyse aux rayons X de poudres de l'échantillon B entre $2\theta=1°$ et $2\theta=24°$ obtenues à l'aide d'une radiation Mo-K$\alpha$ ($\lambda$ = 0,71073Å).

La **figure 3C** présente le spectre d'analyse aux rayons X de poudres de l'échantillon C entre $2\theta=1°$ et $2\theta=24°$ obtenues à l'aide d'une radiation Mo-K$\alpha$ ($\lambda$ = 0,71073Å).

L'échantillon A correspond à la poudre de sel de formule (I) $(HMTBA)_2Ca$ obtenu dans la première partie de l'exemple 9.

L'échantillon B correpond à la poudre obtenue à l'issue de l'exemple 1.

L'échantillon C correpond à la poudre obtenue à l'issue de l'exemple 12.

La **figure 4** présente une analyse au microscope électronique à balayage (MEB) couplée à de la spectrométrie d'émission X, réalisée au cœur (A) et à la surface (B) d'une même particule selon l'exemple 3.

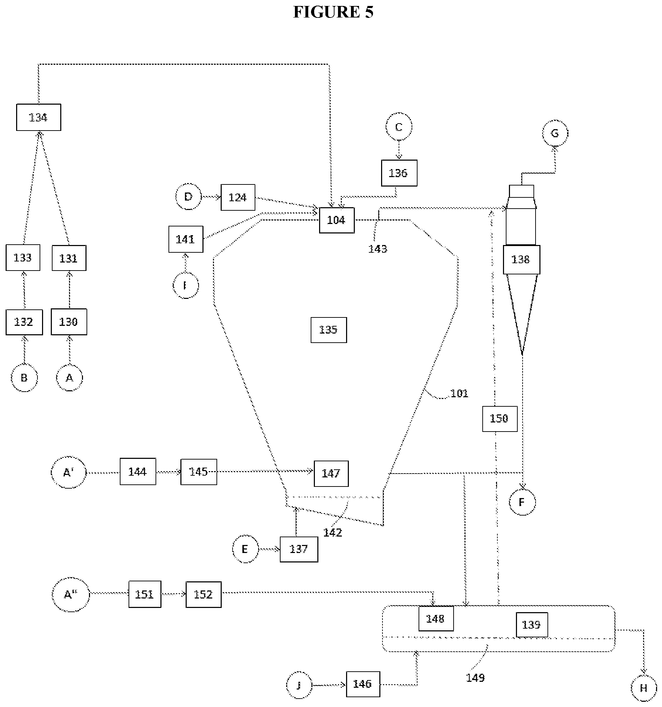

La **figure 5** est un schéma de principe d'un procédé selon l'invention, mis en œuvre dans une tour multiples effets. Un milieu aqueux contenant un acide, symbolisé par le cercle A, traverse éventuellement un réchauffeur 130 et alimente par une pompe 131 le dispositif de mise en contact 134. Un milieu aqueux contenant un métal ou cation métallique symbolisé par le cercle B traverse éventuellement un réchauffeur 132 et alimente par une pompe 133 le dispositif de mise en contact 134. La phase aqueuse résultant du mélange entre le milieu aqueux A et le milieu aqueux B, est pulvérisée dans la tour d'atomisation via le dispositif de pulvérisation 104 destiné à la production d'aérosols monodisperses ou polydisperses.

Un milieu aqueux contenant un acide, symbolisé par le cercle A', traverse éventuellement un réchauffeur 144 et alimente par une pompe 145 le dispositif de pulvérisation 147 destiné à la production d'aérosols.

Un milieu aqueux contenant un acide, symbolisé par le cercle A", traverse éventuellement un réchauffeur 151 et alimente par une pompe 152 le dispositif de pulvérisation 148 destiné à la production d'aérosols.

Le cercle C représente un dispositif de pulvérisation complémentaire d'agent antiagglomérant via un doseur de poudre 136, si nécessaire.

Le cercle D représente l'introduction du gaz vecteur chaud, **notamment air et/ou gaz inerte,** en version séchage par atomisation, via le ventilateur 124.

Le cercle E représente l'introduction du gaz vecteur secondaire, pour le séchage ou /et le refroidissement final de la composition finale stabilisée obtenue, solide ou en cours de solidification via un ventilateur 137.

Le cercle J représente l'introduction du gaz vecteur sur le lit fluidisé vibré externe 139, pour le séchage ou /et le refroidissement final de la composition finale stabilisée obtenue, solide ou en cours de solidification via le ventilateur 146.

Un cyclone 138 sépare tout ou partie du produit final F c'est-à-dire la composition pulvérulente, que l'on récupère, et le gaz vecteur G que l'on évacue.

Le lit fluidisé vibré externe 139 permet la récupération de tout ou partie du produit final H, c'est-à-dire la composition pulvérulente, par le bas de la tour.

L'introduction de l'air secondaire E a lieu à travers un fond perméable 142 de la tour 135 pour mettre la matière pulvérulente sous forme de lit fluidisé. L'air usé est évacué par un ou plusieurs orifice(s) 143 prévu(s) à travers la paroi supérieure de l'enceinte 101.

L'introduction de l'air secondaire J a lieu à travers un fond perméable 149 du lit fluidisé vibré 139 pour mettre la matière pulvérulente sous forme de lit fluidisé. L'air usé est évacué par la ligne 150 raccordée sur l'entrée du cyclone 138.

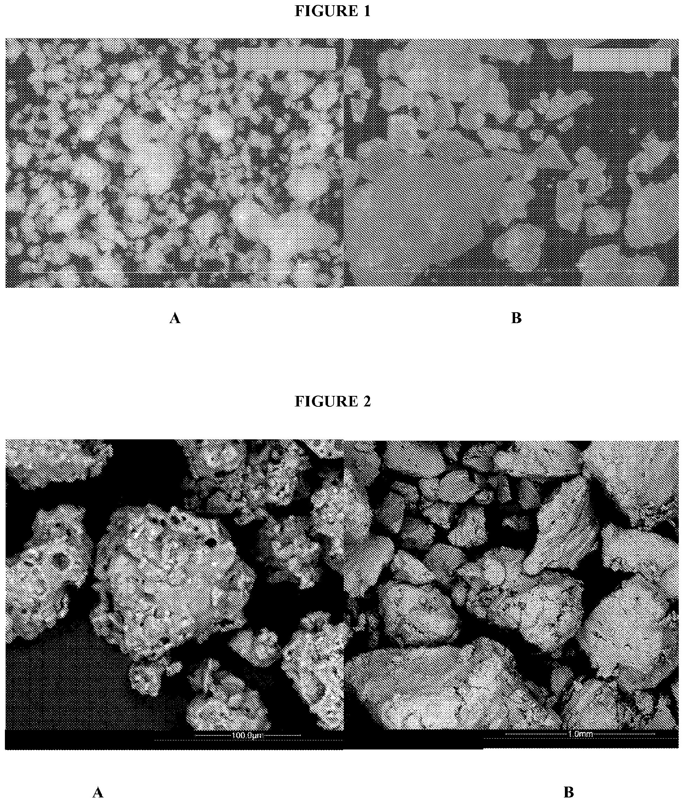

Dans cet exemple, l'air usé passe ensuite par le cyclone 138 qui produit d'un côté des particules de produit F et de l'autre de l'air à évacuer G. La majeure partie des particules est recueillie juste au-dessus de la paroi perméable 142. La figure 1 illustre que les particules sont collectées soit directement en F, soit par l'intermédiaire du lit fluidisé externe 139 en H.

Il peut en outre être prévu l'addition, représentée par le cercle I, en zone de pulvérisation, d'une substance en poudre, en particulier des fines particules de la composition pulvérulente récupérées en sortie du cyclone 138, produit F, ou de l'installation, injectée au moyen du dispositif 141 constitué principalement d'un doseur de poudre.

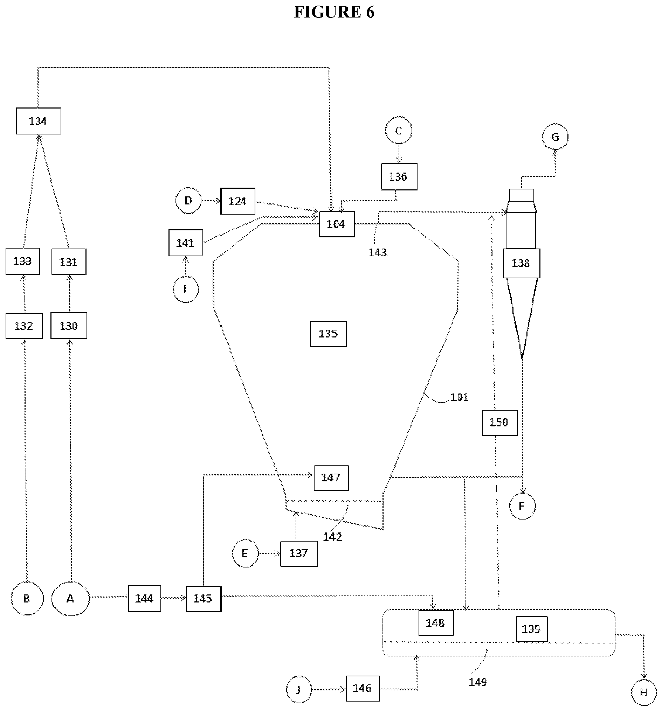

La **figure 6** est un schéma de principe d'un procédé selon l'invention, mis en œuvre dans une tour multiples effets et tel que décrit dans la **figure 5,** avec A = A' = A".

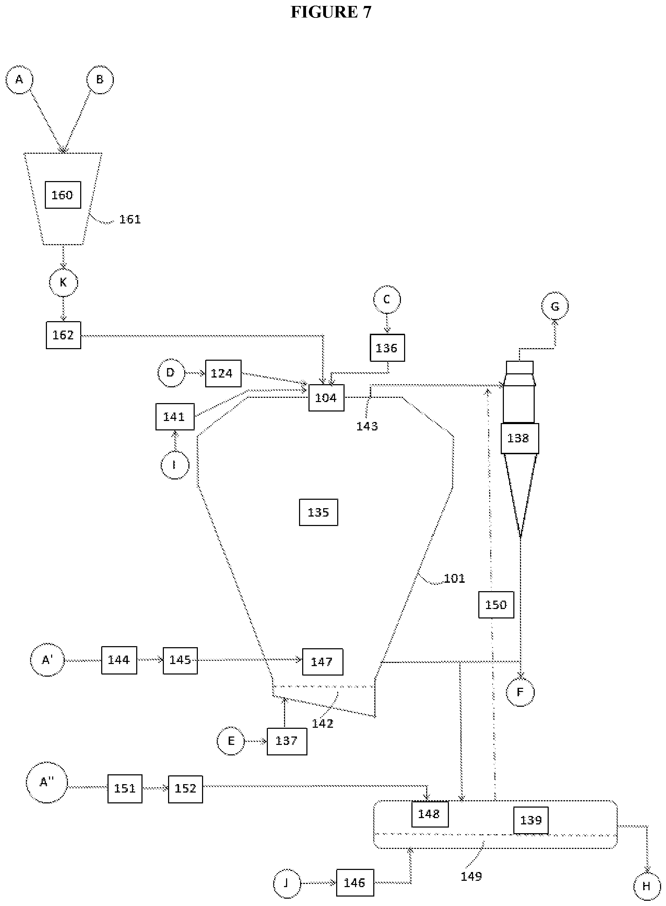

La **figure 7** est un schéma de principe d'un procédé selon l'invention, mis en œuvre dans une tour multiples effets.

Un milieu aqueux contenant un acide, symbolisé par le cercle A est transféré dans un réacteur C équipé d'une double enveloppe thermostatée 161. Un milieu aqueux contenant un métal ou cation métallique symbolisé par le cercle B est ajouté progressivement dans le réacteur C sous agitation. La phase aqueuse K résultant du mélange entre le milieu aqueux A et le milieu aqueux B, est alimentée par la pompe d'envoi 162 pour être pulvérisée dans la tour d'atomisation via le dispositif de pulvérisation 104 destiné à la production d'aérosols monodisperses ou polydisperses.

Un milieu aqueux contenant un acide, symbolisé par le cercle A', traverse éventuellement un réchauffeur 144 et alimente par une pompe 145 le dispositif de pulvérisation 147 destiné à la production d'aérosols.

Un milieu aqueux contenant un acide, symbolisé par le cercle A", traverse éventuellement un réchauffeur 151 et alimente par une pompe 152 le dispositif de pulvérisation 148 destiné à la production d'aérosols.

Le cercle C représente un dispositif de pulvérisation complémentaire d'agent antiagglomérant via un doseur de poudre 136, si nécessaire.

Le cercle D représente l'introduction du gaz vecteur chaud, notamment air et/ou gaz inerte, en version séchage par atomisation, via le ventilateur 124.

Le cercle E représente l'introduction du gaz vecteur secondaire, pour le séchage ou /et le refroidissement final de la composition finale stabilisée obtenue, solide ou en cours de solidification via un ventilateur 137.

Le cercle J représente l'introduction du gaz vecteur sur le lit fluidisé vibré externe 139, pour le séchage ou /et le refroidissement final de la composition finale stabilisée obtenue, solide ou en cours de solidification via le ventilateur 146.

Un cyclone 138 sépare tout ou partie du produit final F c'est-à-dire la composition pulvérulente, que l'on récupère, et le gaz vecteur G que l'on évacue.

Le lit fluidisé vibré externe 139 permet la récupération de tout ou partie du produit final H, c'est-à-dire la composition pulvérulente, par le bas de la tour.

L'introduction de l'air secondaire E a lieu à travers un fond perméable 142 de la tour 135 pour mettre la matière pulvérulente sous forme de lit fluidisé. L'air usé est évacué par un orifice 143 prévu à travers la paroi supérieure de l'enceinte 101.

L'introduction de l'air secondaire J a lieu à travers un fond perméable 149 du lit fluidisé vibré 139 pour mettre la matière pulvérulente sous forme de lit fluidisé. L'air usé est évacué par la ligne 150 raccordée sur l'entrée du cyclone 138.

Dans cet exemple, l'air usé passe ensuite par le cyclone 138 qui produit d'un côté des particules de produit F et de l'autre de l'air à évacuer G. La majeure partie des particules est recueillie juste au-dessus de la paroi perméable 142. La figure 1 illustre que les particules sont collectées soit directement en F, soit par l'intermédiaire du lit fluidisé externe 139 en H.

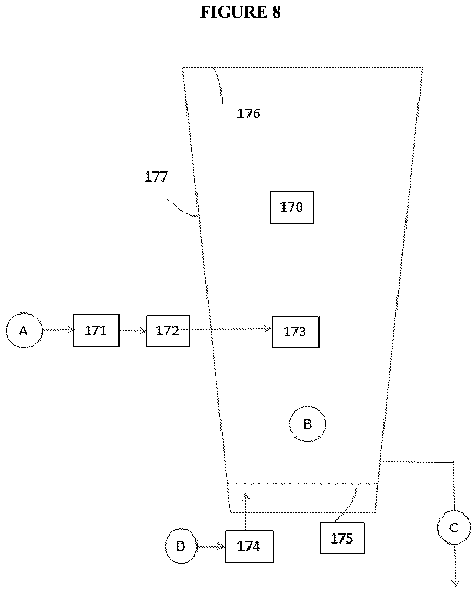

Il peut en outre être prévu l'addition, représentée par le cercle I, en zone de pulvérisation, d'une substance en poudre, en particulier des fines particules de la composition pulvérulente récupérées en sortie du cyclone 138, produit F, ou de l'installation, injectée au moyen du dispositif 141 constitué principalement d'un doseur de poudre. La **figure 8** est un schéma de principe d'un procédé selon l'invention, mis en œuvre dans un lit d'air fluidisé.

Une composition pulvérulente de type sel (HMTBA)$_2$Ca, symbolisé par le cercle B est incorporée dans un lit d'air fluidisé 170. Un milieu aqueux contenant un acide, symbolisé par le cercle A, traverse éventuellement un réchauffeur 171 et alimente par une pompe 172 un dispositif de pulvérisation 173 destiné à la production d'aérosols.

Le cercle D représente l'introduction du gaz vecteur, pour le séchage ou /et le refroidissement final de la composition finale stabilisée obtenue, solide ou en cours de solidification via un ventilateur 174.

L'introduction du gaz D a lieu à travers un fond perméable 175 du lit fluidisé pour mettre la matière pulvérulente B sous forme de lit fluidisé. L'air usé est évacué au travers de filtre(s) par un orifice 176 prévu à travers la paroi supérieure de l'enceinte 177.

La composition pulvérulente finale H est récupérée en fin de batch lors de la vidange du lit fluidisé.

## EXEMPLES

Les exemples 1 à 11 qui suivent illustrent l'invention. L'exemple 12 est un exemple comparatif n'appartenant pas à la présente invention.

**Exemple 1 :** Préparation d'une poudre ayant un TOS de 88,3% en masse

Un kilogramme de sel (HMTBA)$_2$Ca à 85,5 % de TOS, 11,7 % de calcium et 2,3 % d'humidité a été incorporé dans un lit d'air fluidisé d'un volume utile de 5 litres. Sur cette poudre, 300 g d'une solution d'HMTBA à 88% de matière sèche a été pulvérisée à un débit de 450 g/h, une pression de pulvérisation de 1,5 bars et une température d'entrée sur le lit d'air fluidisé de 60 °C. A l'issu de la pulvérisation, le produit a été séché durant 5 min.

Le produit obtenu a un TOS de 88,3 %, une teneur en calcium de 9,2 % et une humidité de 1,3%. La granulométrie moyenne de ce produit est de 191 μm, la densité non tassée de 390 g/L et la densité tassée à 480 g/L

**Exemple 2 :** Préparation d'une poudre ayant un TOS de 89,3% en masse

Un kilogramme de sel (HMTBA)$_2$Ca à 84,6 % de TOS, 11,5 % de calcium et 1,9 % d'humidité a été incorporé dans un lit d'air fluidisé d'un volume utile de 5 litres. Sur cette poudre, 504 g d'une solution d'HMTBA à 88% de matière sèche a été pulvérisée à un débit de 250 g/H, une pression de pulvérisation de 1,5 bars et une température d'entrée sur le lit d'air fluidisé de 60 °C. A l'issu de la pulvérisation, le produit a été séché durant 5 min.

Le produit obtenu a un TOS de 89,3 %, une teneur en calcium de 8 % et une humidité de 1.6%.

**Exemple 3 :** Préparation d'une poudre ayant un TOS de 88,2% en masse

Deux kilogrammes de sel (HMTBA)$_2$Ca à 85,5 % de TOS, 11,7 % de calcium et 2,3 % d'humidité ont été incorporés dans un lit d'air fluidisé d'un volume utile de 12 litres. Sur cette poudre, 670 g d'une solution d'HMTBA à 88% de matière sèche a été pulvérisée à un débit de 600 g/H, une pression de pulvérisation de 1 bars et une température d'entée du lit d'air fluidisé de 55 °C. A l'issu de la pulvérisation, le produit a été séché durant 5 min.

Le produit obtenu a un TOS de 88,2 %, une teneur en calcium de 8,8 %, et une humidité de 2,2 %. La granulométrie moyenne de ce produit est de 150 μm, la densité non tassée de 370 g/L, et la densité tassée de 400 g/L.

**Exemple 4 :** Préparation d'une poudre ayant un TOS de 88,1% en masse

Une étape de broyage sur un broyeur à couteau est réalisée sur deux kilogrammes d'un sel (HMTBA)$_2$Ca sous

forme d'extrudés obtenu selon le brevet FR2964968. La poudre obtenue post broyage a un TOS de 74 %, une teneur en calcium de 11,2 %, une teneur en eau de 11 % et une granulométrie moyenne de 150 $\mu$m. Un kilogramme de ce produit est incorporé dans un lit d'air fluidisé d'un volume utile de 5 litres. Sur cette poudre, 400 g d'une solution d'HMTBA à 88 % de matière sèche est pulvérisée à un débit de 300 g/H, une pression de pulvérisation de 1,5 bars et une température d'entrée du lit d'air fluidisé de 60 °C. Le produit est ensuite séché durant 30 min.

Le produit obtenu a un TOS de 88,1 %, une teneur en calcium de 9,2 %, et une humidité de 1,4 %. La granulométrie moyenne de ce produit est de 250 $\mu$m et la densité non tassée de 510 g/L.

**Exemple 5 :** Préparation d'une poudre ayant un TOS de 88,4% en masse

Un kilogramme de sel (HMTBA)$_2$Ca à 85,5 % de TOS, 11,7 % de calcium et 2,3 % d'humidité est incorporé dans un lit d'air fluidisé. Sur cette poudre, 275 g d'une solution d'HMTBA à 95,47% de matière sèche chauffée à une température de 60 °C est pulvérisée à un débit de 300 g/H, une pression de pulvérisation de 1,5 bars et une température d'entée du lit d'air fluidisé de 60 °C. Le produit est ensuite séché durant 5 min.

Le produit obtenu a un TOS de 88,4 %, une teneur en calcium de 8,9 %, et une humidité de 1,5 %. La granulométrie moyenne de ce produit est de 680 $\mu$m, la densité non tassée de 380 g/L, et la densité tassée de 410 g/L.

**Exemple 5 bis :** Préparation d'une poudre ayant un TOS de 88,2% en masse

Trois kilogrammes de sel (HMTBA)$_2$Ca à 85,5 % de TOS, 11,7 % de calcium et 2,3 % d'humidité sont incorporés dans un granulateur rotatif d'un volume utile de 5 litres de type GLATT GRC3. Sur cette poudre, 1 kg d'une solution d'HMTBA à 88 % de matière sèche est pulvérisée à un débit de 600 g/H, une pression de pulvérisation de 1,5 bars, une température d'entrée du granulateur de 60 °C et une vitesse de rotation du disque de 200 tours/min. Le produit est ensuite séché durant 5 min.

Le produit obtenu a un TOS de 88,2 %, une teneur en calcium de 9,1 % et une humidité de 1,7 %. La granulométrie moyenne de ce produit est de 230 $\mu$m et la densité non tassée de 540 g/L.

**Exemple 6 :** Préparation d'une poudre ayant un TOS de 88,1% en masse

Une poudre de sel (HMTBA)$_2$Ca à 85,2 % de TOS, 11,8 % de calcium et 1,8 % d'humidité a été alimentée en continu une tour d'atomisation multiple effet à un débit de 200 kg/H. En partie basse de la tour de séchage a été pulvérisée de manière continue une solution d'HMTBA à 88 % de matière sèche. Cette solution a été pulvérisée d'une part sur le lit statique de l'installation industrielle à un débit de 60kg/h et une pression de pulvérisation de 4 bars, et d'autre part sur le vibrofluidiseur à un débit de 16 kg/H et une pression de pulvérisation de 1,5 bars.

Les températures appliquées ont été de 100 °C pour la température du lit statique, 70 °C pour la première partie du vibrofluidiseur et 30 °C pour la seconde partie du vibrofluidiseur.

Le produit obtenu a un TOS de 88,1 %, une teneur en calcium de 9,1 %, et une humidité de 1,4 %. La granulométrie moyenne de ce produit est de 196 $\mu$m, la densité non tassée de 530 g/L et la densité tassée à 10 coups de 560 g/L.

**Exemple 7 :** Préparation d'une poudre ayant un TOS de 88,5% en masse

Une poudre de sel (HMTBA)$_2$Ca à 85,18 % de TOS, 11,78 % de calcium et 1,79 % d'humidité est alimentée en continu dans une tour multiple effet à un débit de 200 kg/H. En partie basse de la tour de séchage est pulvérisée de manière continue une solution d'HMTBA à 96 % de matière sèche, chauffée à une température de 60 °C de manière à abaisser sa viscosité en dessous de 200 centipoises. Cette solution est pulvérisée d'une part sur le lit statique de l'installation industrielle à un débit de 48 kg/h et une pression de pulvérisation de 3,5 bars, et d'autre part sur le vibrofluidiseur à un débit de 13 kg/H et une pression de pulvérisation de 1,5 bars.

Les températures des lignes d'alimentation liquides sont thermostatées à 60 °C de manière à assurer une pulvérisation satisfaisante de la solution d'HMTBA concentrée.

Les températures appliquées sont de 100 °C pour la température du lit statique, 70 °C pour la première partie du vibrofluidiseur et 30 °C pour la seconde partie du vibrofluidiseur.

Le produit obtenu a un TOS de 88,5%, une teneur en calcium de 8,8 %, et une humidité de 1,3 %. La granulométrie moyenne de ce produit est de 250 $\mu$m et la densité non tassée de 550 g/L.

**Exemple 8 :** Préparation d'une poudre ayant un TOS de 88,3% en masse

Un lait de chaux préparé à 30 % de matière sèche et une solution d'HMTBA à 88 % de matière sèche sont mélangés en continu selon les conditions du brevet FR2988091.

Les débits d'alimentation sont respectivement de 95 kg/H pour le lait de chaux et 130 kg/H pour la solution d'HMTBA.

Le mélange réactionnel est pulvérisé à l'aide d'une buse selon la connaissance de l'homme de l'art, dans une tour d'atomisation multiple effet avec une température d'entrée de 180 °C et une température de sortie de 102 °C.

En bas de tour, une solution d'HMTBA à 88 % d'EST est pulvérisée d'une part sur le lit statique de la tour MSD à un débit de 35 kg/h et une pression de pulvérisation de 3 bars, et d'autre part sur le vibrofluidiseur à un débit de 10 kg/H et une pression de pulvérisation de 1,5 bars.

Les températures appliquées sont de 70 °C pour la température du lit statique, 60 °C pour la première partie du vibrofluidiseur et 30 °C pour la seconde partie du vibrofluidiseur.

Le produit obtenu a un TOS de 88,3 %, une teneur en calcium de 8,9 %, et une humidité de 1,6%. La granulométrie moyenne de ce produit est de 180 $\mu$m et la densité non tassée de 420 g/L.

**Exemple 9 :** Préparation d'une poudre ayant un TOS de 88,6% en masse

Un lait de chaux préparé à 30 % de matière sèche et une solution d'HMTBA à 88 % de matière sèche ont été mélangés en continu dans une turbine d'atomisation (de type NIRO Atomiseur). Les débits d'alimentation ont respectivement été de 3,5 kg/H pour le lait de chaux et 4,5 kg/H pour la solution d'HMTBA.

Le mélange réactionnel a été atomisé dans une tour d'atomisation simple effet à une température d'entrée de 140 °C température de sortie de 85 °C.

Le produit a ensuite été repris en lit d'air fluidisé pour simuler une tour multiple effet.

330 g d'une solution d'HMTBA à 88% de matière sèche a été pulvérisée sur 1 kg de poudre fabriquée précédemment à un débit de 300 g/H, une pression de pulvérisation de 1,5 bars et une température d'entée du lit d'air fluidisé de 60 °C. A l'issu de la pulvérisation, le produit a été séché durant 5 min.

Le produit obtenu a un TOS de 88,6 %, une teneur en calcium de 8,7 % et une humidité de 1,5%.

**Exemple 10** : Préparation d'une poudre ayant un TOS de 88,6% en masse

Un lait de chaux préparé à 37 % de matière sèche et une solution d'HMTBA à 88 % de matière sèche sont mélangés en continu selon les conditions du brevet FR2988091.

Les débits d'alimentation sont respectivement de 90 kg/H pour le lait de chaux et 150 kg/H pour la solution d'HMTBA.

Le mélange est pulvérisé à l'aide d'une buse selon la connaissance de l'homme de l'art dans une tour d'atomisation multiple effet avec une température d'entrée de 180 °C et une température de sortie de 105 °C.

En bas de tour, une solution d'HMTBA concentrée à 96 % de matière sèche est pulvérisée d'une part sur le lit statique de la tour MSD à un débit de 31 kg/h et une pression de pulvérisation de 3 bars, et d'autre part sur le vibrofluidiseur à un débit de 16 kg/H et une pression de pulvérisation de 1,5 bars.

Les températures des lignes d'alimentation liquides sont thermostatées à 60 °C.

Les températures appliquées sont de 70 °C pour la température du lit statique, 60 °C pour la première partie du vibrofluidiseur et 30 °C pour la seconde partie du vibrofluidiseur.

Le produit obtenu a un TOS de 88,6%, une teneur en calcium de 8,7%, et une humidité de 1,3%. La granulométrie moyenne de ce produit est de 210 $\mu$m et la densité non tassée de 430 g/L.

**Exemple 11 :** Préparation d'une poudre ayant un TOS de 88,2% en masse

Un lait de chaux préparé à 30 % de matière sèche et une solution d'HMTBA à 88 % de matière sèche sont mélangés en continu selon les conditions du brevet FR2988091.

Les débits d'alimentation sont respectivement de 75 kg/H pour le lait de chaux et 102 kg/H pour la solution d'HMTBA.

Le mélange réactionnel est pulvérisé à l'aide d'une buse selon la connaissance de l'homme de l'art, dans une tour d'atomisation multiple effet avec une température d'entrée de 160 °C et une température de sortie de 85 °C. Le séchage est réalisé sous azote dans un tour équipée d'un système de recyclage du gaz en circuit fermé.

En bas de tour, une solution d'HMTBA à 88 % d'EST est pulvérisée d'une part sur le lit statique de la tour MSD à un débit de 20 kg/h et une pression de pulvérisation de 3 bars, et d'autre part sur le vibrofluidiseur à un débit de 15 kg/H et une pression de pulvérisation de 1,5 bars.

Les températures appliquées sont de 60 °C pour la température du lit statique, 50 °C pour la première partie du vibrofluidiseur et 20 °C pour la seconde partie du vibrofluidiseur.

Le produit obtenu a un TOS de 88,2 %, une teneur en calcium de 9 %, et une humidité de 1 %. La granulométrie moyenne de ce produit est de 240 μm et la densité non tassée de 480 g/L.

**Exemple 12** : Préparation d'une poudre comprenant du HMTBA selon un procédé n'appartenant pas à la présente invention, et comparaison du produit obtenu avec un produit selon l'invention

La préparation s'est opérée en batch dans un mélangeur à bras en Z ouvert à l'atmosphère. 372 g d'une poudre d'HMTBA$_2$(Ca) cristalline a été incorporée dans le mélangeur puis chauffée à 85 °C grâce à la double enveloppe du matériel.

Une solution d'HMTBA à 88 % de matière sèche a été ajoutée à 4 reprises et à intervalles de 15 min dans le mélangeur en fonctionnement. Les quantités ajoutées ont été de 93 g, 92 g, 94 g et 96 g. A l'issu du dernier ajout la préparation a été maintenue sous agitation durant 37 min à une température de 73-82 °C. La pâte récupérée a ensuite subi une opération de séchage pendant 24 h à l'étuve à 70 °C.

Le produit obtenu post séchage a ensuite été broyé de manière à obtenir des particules grossières.

Une analyse comparative des produits fabriqués selon l'exemple 12 et selon l'exemple 1 de la présente demande a été réalisée.

Le tableau ci-dessous indique les propriétés physiques et chimiques de poudres.

| | **Exemple 1**<br>(Procédé appartenant à la présente invention) | **Exemple 12**<br>(Procédé n'appartenant pas à la présente invention) |
|---|---|---|
| Propriétés physiques | | |
| Granulométrie, μm | Produit homogène en granulométrie Courbe de Gauss avec une médiane à environ 200 μm | Produit hétérogène en granulométrie avec des agglomérats qui font de quelques mm à plusieurs dizaine de cm |
| Densité non tassée, g/l | 370 | 680 |
| Propriétés chimiques | | |
| Humidité, % | 1,5 | 1 |
| Calcium, % | 10,1 | 9,4 |
| TOS, % | 88,4 | 89,9 |

Ces résultats indiquent très clairement un écart significatif en ce qui concerne les propriétés physiques de ces poudres. L'exemple 12 conduit à l'obtention de granulés hétérogènes en taille avec une densité > 650 g/l alors que l'exemple 1 de la présente demande conduit à l'obtention d'une poudre homogène en taille avec une densité proche de 400 g/l.

D'autres analyses permettent de distinguer les deux types de produits. Ainsi l'aspect visuel des poudres a été étudié au microscope optique (figure 1) et au microscope optique à balayage (figure 2).

Les particules fabriquées selon l'exemple 1 sont de petites particules sphériques, avec une distribution granulométrique assez resserrée et de couleur crème.

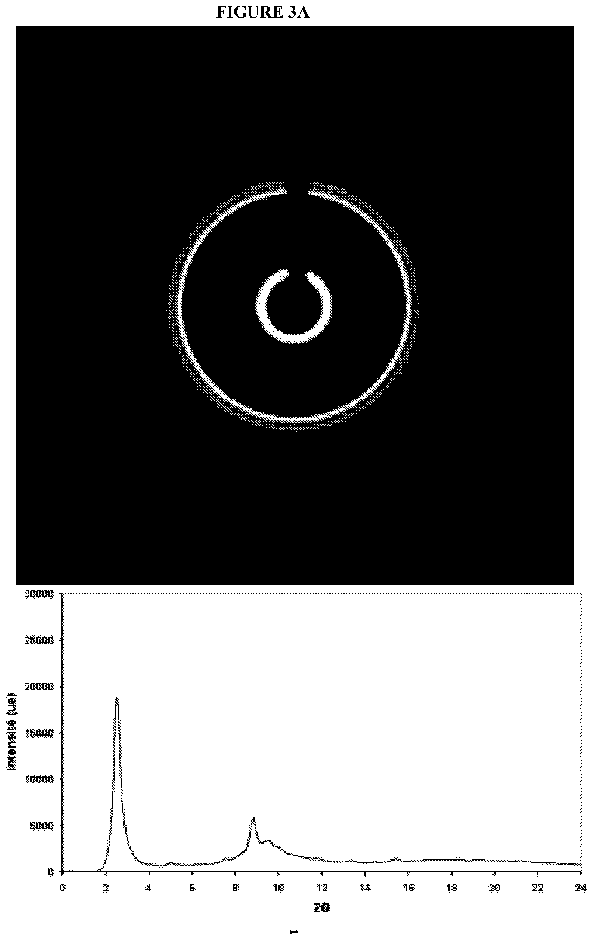

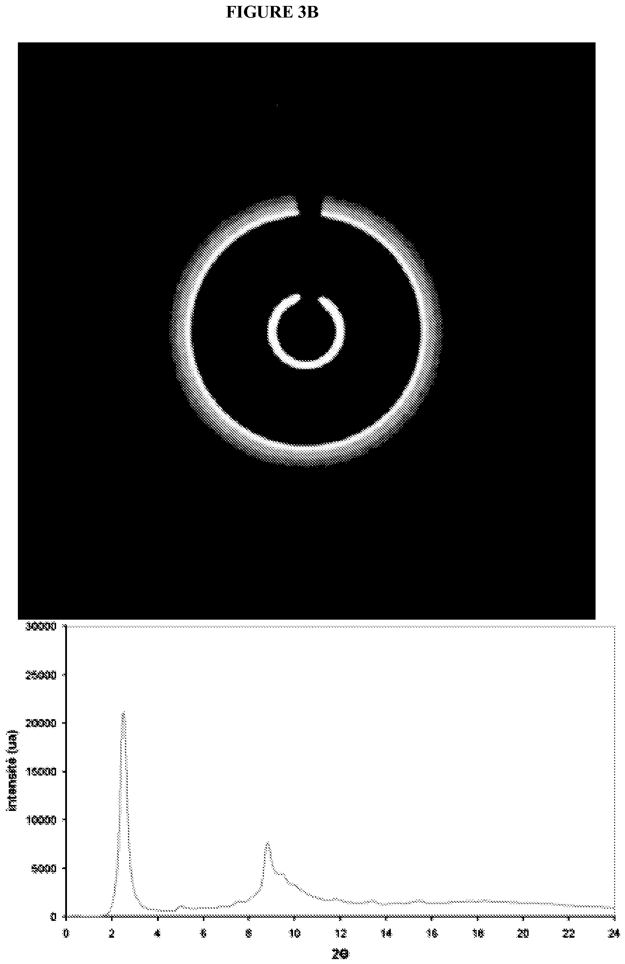

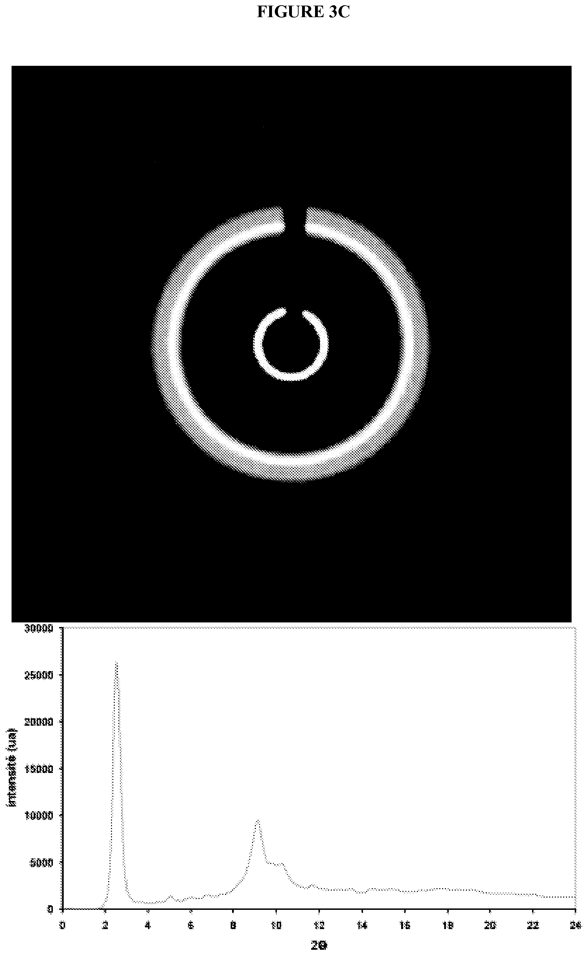

Les particules de l'exemple 12 sont des agrégats compacts qui peuvent être anguleux, hétérogènes en taille et en forme, de couleur brune et avec un aspect lisse en surface. L'analyse aux rayons X a permis également de mettre en évidence des différences en ce qui concerne le degré de cristallinité des particules obtenues selon le procédé (figure 3), liée à l'intensité du pic à 2théta=9°.

Ces résultats permettent de proposer un classement par ordre croissant de cristallinité
A < B < C.

L'échantillon A correspond à la poudre de sel de formule (I) (HMTBA)$_2$Ca obtenu dans l'exemple 9, avant pulvérisation : il s'agit du noyau de sel de formule (I) (HMTBA)$_2$Ca, sans couche extérieure.

L'échantillon B correpond à la poudre obtenue à l'issue de l'exemple 1.

L'échantillon C correpond à la poudre obtenue à l'issue de l'exemple 12.

La poudre A (noyau de sel de formule (I) sans couche extérieure) et la poudre B (objet de la présente invention), sont donc moins cristallines que la poudre C.

**Exemple 13 :** Analyse MEB couplée à de la spectrométrie d'émission X des particules obtenues selon l'exemple 3

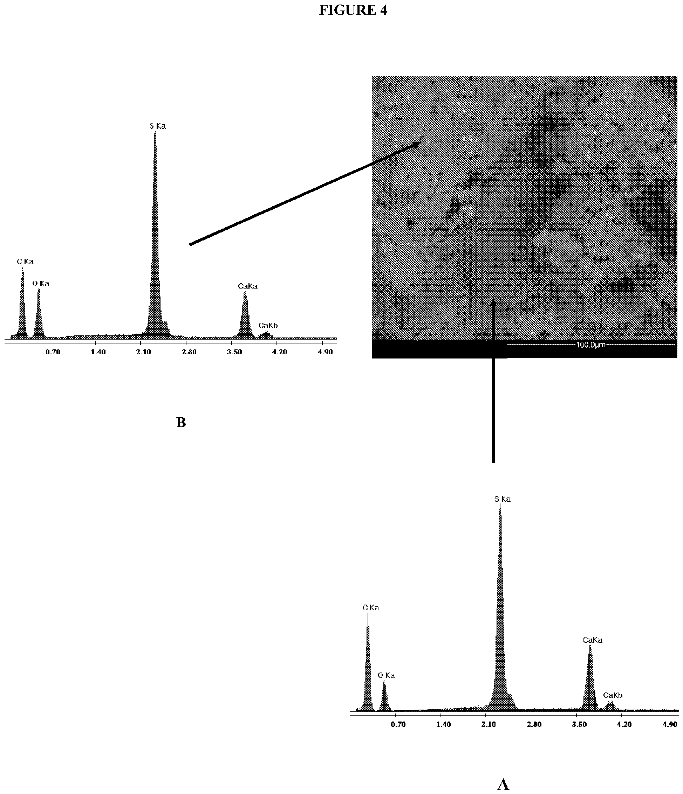

Une analyse MEB couplée à de la spectrométrie d'émission X a été réalisée sur une particule fabriquée selon l'exemple 3 de manière à mettre en évidence la différence de composition chimique entre le cœur de l'extérieur de la particule (figure 4).
La quantification intérieure (figure 4A) est la suivante :

| Elément | % Masse | % At |
|---------|---------|------|
| C | 44,11 | 62,47 |
| O | 17,88 | 19,01 |
| S | 22,58 | 11,98 |
| Ca | 15,42 | 6,54 |
| | | |
| Total | 100,00 | 1 00,00 |

Le rapport atomique S/Ca est, à l'intérieur de la particule (dans le noyau), d'environ 1,8.
La quantification extérieure (figure 4B) est la suivante :

| Elément | % Masse | % At |
|---------|---------|------|
| C | 53,01 | 67,39 |
| O | 22,74 | 21,70 |
| s | 17,60 | 8,38 |
| Ca | 6,65 | 2,53 |
| | | |
| Total | 100,00 | 100,00 |

Le rapport atomique S/Ca est, à l'extérieur de la particule (à sa surface), d'environ 3,3. Cette analyse permet de mettre en évidence une différence de composition chimique entre le cœur de la particule et l'extérieur de la particule notamment en ce qui concerne le pourcentage en calcium
La composition chimique théorique du sel de formule (I) $(HMTBA)_2Ca$ et du complexe de formule (II) $(HMTBA)_4Ca$ est la suivante :

**Forme sel de formule (I) = 338 g/ mol**

| Atomes | Nombres | Poids moléculaire total, g/mol | % sur poids moléculaire de la molécule considérée |
|--------|---------|-------------------------------|---------------------------------------------------|
| Carbone | 10 | 120 | 35.5 |
| Oxygène | 6 | 96 | 28.4 |
| Soufre | 2 | 64 | 18.9 |
| Hydrogène | 18 | 18 | 5.32 |
| Calcium | 1 | 40 | 11.8 |

Le rapport atomique théorique S/Ca est, pour le sel de formule (I), d'environ 1,6.

**Forme complexe de formule (II)= 636 g/ mol**

| Atomes | Nombres | Poids moléculaire total, g/mol | % sur poids moléculaire de la molécule considérée |
|--------|---------|-------------------------------|---------------------------------------------------|
| Carbone | 20 | 240 | 37.7 |
| Oxygène | 12 | 192 | 30.1 |
| Soufre | 4 | 128 | 20.1 |

(suite)

| Atomes | Nombres | Poids moléculaire total, g/mol | % sur poids moléculaire de la molécule considérée |
|---|---|---|---|
| Hydrogène | 36 | 36 | 5.6 |
| Calcium | 1 | 40 | 6.28 |

Le rapport atomique théorique S/Ca est, pour le sel de formule (I), d'environ 3,2.

La comparaison entre les valeurs théoriques et les valeurs mesurées en ce qui concerne les % en soufre et le calcium indique la présence d'un sel de formule (I) d'HMBTA à l'intérieur de la particule et d'un complexe de formule (II) à l'extérieur.

## Revendications

1. Particule comprenant :

   • un noyau constitué essentiellement d'un sel de formule (I) suivante :

   $$(A^-)_n M^{n+} \qquad (I)$$

   dans laquelle :

   $A^-$ représente un anion choisi dans le groupe constitué du 2-hydroxy-4-méthyl-thio-butanoate, du méthioniate et du cystéinate,
   M représente un métal divalent ou trivalent,
   n étant égal à 2 lorsque ledit métal est divalent et à 3 lorsque ledit métal est trivalent, et

   • une couche comprenant un composé B choisi dans le groupe constitué de l'acide 2-hydroxy-4-méthyl-thio-butanoïque (HMTBA), de ses sels et de ses complexes, et de leurs mélanges,

   ladite couche enrobant ledit noyau,
   le pourcentage massique dudit composé B par rapport au sel de formule (I) du noyau étant compris de 10% à 40%,
   ledit composé B n'étant pas, ou pas uniquement, sous la forme d'un sel de formule (I),
   la teneur en composés organiques soufrés (TOS) de ladite particule étant supérieure à 87% en masse, en particulier supérieure à 88%, 89% ou 90% en masse, par rapport à la masse totale de ladite particule.

2. Particule selon la revendication 1, dans laquelle ledit composé B est sous la forme :

   - libre, choisie parmi l'acide 2-hydroxy-4-méthyl-thio-butanoïque (HMTBA), et/ou
   - dudit sel de formule (I) tel que défini dans la revendication 1, et/ou
   - d'un complexe de formule $(A)_4 M$ (II) dans laquelle A et M sont tels que définis dans la revendication 1, A représentant l'acide 2-hydroxy-4-méthyl-thio-butanoïque (HMTBA),

   ledit composé B n'étant pas, ou pas uniquement, sous la forme d'un sel de formule (I),
   ledit composé B étant en particulier sous la forme :

   - libre,
   - du complexe de formule (II),
   - d'un mélange de la forme libre et du complexe de formule (II),
   - d'un mélange de la forme libre et du sel de formule (I),
   - d'un mélange du sel de formule (I) et du complexe de formule (II), ou
   - d'un mélange de la forme libre, du sel de formule (I) et du complexe de formule (II).

3. Particule selon l'une quelconque des revendications 1 à 2, dans laquelle :

   - la teneur en eau est inférieure à 3%, en particulier moins de 2% ou 1,5%, en masse,

- la teneur en calcium est comprise de 6 à 11% en masse, en particulier de 6,5 à 10%, plus particulièrement de 7 à 9%, la teneur en calcium étant notamment de 8%, ou
- le pourcentage massique dudit composé B par rapport au sel de formule (I) du noyau est compris de 10% à 40%, en particulier de 15 % à 35%, plus particulièrement de 20% à 32%.

4. Particule selon l'une quelconque des revendications 1 à 3, dans laquelle ledit métal est choisi dans le groupe comprenant Mg, Be, Ca, Sr, Ba, Mn, Fe, Co, Ni, Cu, Zn, Pt, B, Al, Ga, In, en particulier Mg, Ca, Fe, Mn, Al, Cu, Zn, ledit sel de formule (I) étant notamment $(HMTBA)_2Ca$, $(HMTBA)_2Mg$, $(HMTBA)_2Fe$, $(HMTBA)_2Mn$, $(HMTBA)_2Zn$, $(HMTBA)_2Cu$, $(HMTBA)_3Fe$, $(HMTBA)_3Al$, $(Méthionine)_2Ca$, $(Méthionine)_2Mg$, $(Méthionine)_2Fe$, $(Méthionine)_2Mn$, $(Méthionine)_2Zn$, $(Méthionine)_2Cu$, $(Méthionine)_3Fe$, $(Méthionine)_3Al$, $(Cystéine)_2Ca$, $(Cystéine)_2Mg$, $(Cystéine)_2Fe$, $(Cystéine)_2Mn$, $(Cystéine)_2Zn$, $(Cystéine)_2Cu$, $(Cystéine)_3Fe$, ou $(Cystéine)_3Al$, encore plus particulièrement un sel de formule $(HMTBA)_2Ca$, $(HMTBA)_2Mg$, $(HMTBA)_2Fe$, $(HMTBA)_2Mn$, $(HMTBA)_2Zn$, $(HMTBA)_2Cu$, $(Méthionine)_2Ca$, $(Méthionine)_2Mg$, $(Méthionine)_2Fe$, $(Méthionine)_2Mn$, $(Méthionine)_2Zn$, $(Méthionine)_2Cu$, $(Cystéine)_2Ca$, $(Cystéine)_2Mg$, $(Cystéine)_2Fe$, $(Cystéine)_2Mn$, $(Cystéine)_2Zn$ ou $(Cystéine)_2Cu$.

5. Particule selon l'une quelconque des revendications 1 à 3, dans laquelle :

- ledit anion A⁻ est le 2-hydroxy-4-méthyl-thio-butanoate,
et/ou
- ledit composé B compris dans ladite couche est l'acide 2-hydroxy-4-méthyl-thio-butanoïque (HMTBA), ou l'un de ses sels ou complexes.

6. Composition pulvérulente constituée ou comprenant des particules telles que définies dans l'une quelconque des revendications 1 à 5.

7. Composition pulvérulente de particules selon la revendication 6, dans laquelle la taille granulométrique desdites particules varie de 10 à 3000 μm, en particulier de 20 à 300 μm, plus particulièrement de 100 à 250 μm, en moyenne granulométrique [Dv(0,5)].

8. Composition pulvérulente selon l'une quelconque des revendications 6 à 7, dont :

- la masse volumique non tassée est supérieure à 350 g/L, en particulier supérieure à 400 g/L, ou
- la masse volumique tassée est supérieure à 400 g/L, en particulier supérieure à 450 g/L.

9. Composition pulvérulente selon l'une quelconque des revendications 6 à 8, comprenant, outre lesdites particules, de l'huile, notamment de l'huile végétale.

10. Procédé de préparation d'une particule comprenant :

• un noyau constitué essentiellement d'un sel de formule (I) suivante :

$$(A^-)_n M^{n+} \qquad (I)$$

dans laquelle :

A⁻ représente un anion choisi dans le groupe constitué du 2-hydroxy-4-méthyl-thio-butanoate, du méthioniate et du cystéinate,
M représente un métal divalent ou trivalent,
n étant égal à 2 lorsque ledit métal est divalent et à 3 lorsque ledit métal est trivalent, et

• une couche comprenant un composé B choisi dans le groupe constitué de l'acide 2-hydroxy-4-méthyl-thio-butanoïque (HMTBA),

ladite couche enrobant ledit noyau,
le pourcentage massique dudit composé B par rapport au sel de formule (I) du noyau étant compris de 10% à 50%,
ledit composé B n'étant pas, ou pas uniquement, sous la forme d'un sel de formule (I),
la teneur en composés organiques soufrés (TOS) de ladite particule étant supérieure à 87% en masse, en particulier supérieure à 88%, 89% ou 90% en masse, par rapport à la masse totale de ladite particule,
ledit procédé comprenant une étape de pulvérisation sur un solide constitué essentiellement d'un sel de formule (I) telle que définie précédemment, d'une composition comprenant un composé B choisi dans le groupe constitué de l'acide 2-hydroxy-4-méthyl-thio-butanoïque (HMTBA), la masse dudit composé B étant comprise de 10% à 50% de la masse du sel de formule (I) du solide,
pour obtenir ladite particule,
en particulier, ladite composition comprenant en outre de l'eau, le pourcentage massique en eau de ladite composition étant compris de 0,5 à 50.

**11.** Procédé selon la revendication 10 dans lequel ladite étape de pulvérisation est effectuée :

- en lit d'air fluidisé en discontinu ou en continu,
ou
- sur vibrofluidiseur,
ou
- en tour d'atomisation par copulvérisation,

ladite étape de pulvérisation étant notamment réalisée en continu.

**12.** Procédé selon l'une quelconque des revendications 10 à 11, dans lequel le noyau constitué essentiellement d'un sel de formule (I) est obtenu :

- par atomisation réactive,
- en lit d'air fluidisé, en granulateur, granulateur rotatif, ou en mélangeur,
- par extrusion réactive, ou
- à l'aide d'un mélangeur statique ou dynamique.

**Patentansprüche**

**1.** Partikel, umfassend:

• einen Kern, der im Wesentlichen aus einem Salz mit der folgenden Formel (I) besteht:

$$(A^-)_n M^{n+1} \qquad (I),$$

wobei:

$A^-$ ein Anion darstellt, ausgewählt aus der Gruppe bestehend aus 2-Hydroxy-4-methylthiobutanoat, Methioniat und Cysteinat,
M ein zweiwertiges oder dreiwertiges Metall darstellt,
n gleich 2 ist, wenn das Metall zweiwertig ist, und gleich 3 ist, wenn das Metall dreiwertig ist,

und
• eine Schicht, die eine Verbindung B umfasst, ausgewählt aus der Gruppe bestehend aus 2-Hydroxy-4-methylthiobutansäure (HMTBA), ihren Salzen und ihren Komplexen, und ihren Mischungen,

wobei die Schicht den Kern umhüllt,
wobei der Massenprozentsatz der Verbindung B im Verhältnis zu dem Salz mit der Formel (I) des Kerns von 10 % bis 40 % beträgt,
wobei die Verbindung B nicht, oder nicht nur, in der Form eines Salzes mit der Formel (I) ist,
wobei der Gehalt an organischen Schwefelverbindungen (TOS) des Partikels größer ist als 87 Massen-%, insbesondere größer als 88 Massen-%, 89 Massen-% oder 90 Massen-%, im Verhältnis zur Gesamtmasse des Partikels.

**2.** Partikel nach Anspruch 1, wobei die Verbindung B in der Form ist:

- frei, ausgewählt aus 2-Hydroxy-4-methylthiobutansäure (HMTBA), und/oder
- des Salzes mit der Formel (I), wie in Anspruch 1 definiert, und/oder

- eines Komplexes mit der Formel (A)$_4$M (II), wobei A und M wie in Anspruch 1 definiert sind, wobei A 2-Hydroxy-4-methylthiobutansäure (HMTBA) darstellt,

wobei die Verbindung B nicht, oder nicht nur, in der Form eines Salzes mit der Formel (I) ist, wobei die Verbindung B insbesondere in der Form ist:

- frei,
- des Komplexes mit der Formel (II),
- einer Mischung der freien Form und des Komplexes mit der Formel (II),
- einer Mischung der freien Form und des Salzes mit der Formel (I),
- einer Mischung des Salzes mit der Formel (I) und des Komplexes mit der Formel (II), oder
- einer Mischung der freien Form, des Salzes mit der Formel (I) und des Komplexes mit der Formel (II) .

3. Partikel nach einem der Ansprüche 1 bis 2, wobei:

- der Gehalt an Wasser kleiner ist als 3 Massen-%, insbesondere kleiner als 2 Massen-% oder 1,5 Massen-%,
- der Gehalt an Calcium von 6 bis 11 Massen-% beträgt, insbesondere von 6,5 bis 10 Massen-%, bevorzugt von 7 bis 9 Massen-%, wobei der Gehalt an Calcium vor allem 8 Massen-% beträgt, oder
- der Massenprozentsatz der Verbindung B im Verhältnis zu dem Salz mit der Formel (I) des Kerns von 10 % bis 40 % beträgt, insbesondere von 15 % bis 35 %, bevorzugt von 20 % bis 32 %.

4. Partikel nach einem der Ansprüche 1 bis 3, wobei das Metall ausgewählt ist aus der Gruppe umfassend Mg, Be, Ca, Sr, Ba, Mn, Fe, Co, Ni, Cu, Zn, Pt, B, Al, Ga, In, insbesondere Mg, Ca, Fe, Mn, Al, Cu, Zn, wobei das Salz mit der Formel (I) insbesondere ist: (HMTBA)$_2$Ca, (HMTBA)$_2$Mg, (HMTBA)$_2$Fe, (HMTBA)$_2$Mn, (HMTBA)$_2$Zn, (HMTBA)$_2$Cu, (HMTBA)$_3$Fe, (HMTBA)$_3$Al, (Methionin)$_2$Ca, (Methionin)$_2$Mg, (Methionin)$_2$Fe, (Methionin)$_2$Mn, (Methionin)$_2$Zn, (Methionin)$_2$Cu, (Methionin)$_3$Fe, (Methionin)$_3$Al, (Cystein)$_2$Ca, (Cystein) $_2$Mg, (Cystein)$_2$Fe, (Cystein)$_2$Mn, (Cystein)$_2$Zn, (Cystein)$_2$Cu, (Cystein)$_3$Fe oder (Cystein)$_3$Al, noch bevorzugter ein Salz mit der Formel (HMTBA)$_3$Ca, (HMTBA)$_2$Mg, (HMTBA)$_2$Fe, (HMTBA)$_2$Mn, (HMTBA)$_2$Zn, (HMTBA)$_2$Cu, (Methionin)$_2$Ca, (Methionin) $_2$Mg, (Methionin)$_2$Fe, (Methionin)$_2$Mn, (Methionin)$_2$Zn, (Methionin)$_2$Cu, (Cystein)$_2$Ca, (Cystein)$_2$Mg, (Cystein)$_2$Fe, (Cystein)$_2$Mn, (Cystein)$_2$Zn oder (Cystein)$_2$Cu.

5. Partikel nach einem der Ansprüche 1 bis 3, wobei:

- das Anion A$^-$ 2-Hydroxy-4-methylthiobutanoat ist, und/oder
- die Verbindung B, die in der Schicht enthalten ist, 2-Hydroxy-4-methylthiobutansäure ((HMTBA) ist, oder eines ihrer Salze oder einer ihrer Komplexe.

6. Pulverzusammensetzung, bestehend aus Partikeln oder umfassend Partikel, wie in einem der Ansprüche 1 bis 5 definiert.

7. Pulverzusammensetzung aus Partikeln nach Anspruch 6, wobei die granulometrische Größe der Partikel von 10 bis 3000 $\mu$m variiert, insbesondere von 20 bis 300 $\mu$m, bevorzugt von 100 bis 250 $\mu$m, bezogen auf das ganulometrische Mittel [Dv(0,5)].

8. Pulverzusammensetzung nach einem der Ansprüche 6 bis 7, wobei:

- die nicht verdichtete Volumenmasse größer ist als 350 g/l, insbesondere größer als 400 g/l, oder
- die verdichtete Volumenmasse größer ist als 400 g/l, insbesondere größer als 450 g/l.

9. Pulverzusammensetzung nach einem der Ansprüche 6 bis 8, umfassend, außer den Partikeln, Öl, insbesondere Pflanzenöl.

10. Verfahren zur Herstellung eines Partikels, umfassend:

• einen Kern, der im Wesentlichen aus einem Salz mit der folgenden Formel (I) besteht:

$$(A^-)_n M^{n+1} \qquad (I),$$

wobei:

A⁻ ein Anion darstellt, ausgewählt aus der Gruppe bestehend aus 2-Hydroxy-4-methylthiobutanoat, Methioniat und Cysteinat,
M ein zweiwertiges oder dreiwertiges Metall darstellt,
n gleich 2 ist, wenn das Metall zweiwertig ist, und gleich 3 ist, wenn das Metall dreiwertig ist,

und
• eine Schicht, die eine Verbindung B umfasst, ausgewählt aus der Gruppe bestehend aus 2-Hydroxy-4-methylthiobutansäure (HMTBA),

wobei die Schicht den Kern umhüllt,
wobei der Massenprozentsatz der Verbindung B im Verhältnis zu dem Salz mit der Formel (I) des Kerns von 10 % bis 50 % beträgt,
wobei die Verbindung B nicht, oder nicht nur, in der Form eines Salzes mit der Formel (I) ist,
wobei der Gehalt an organischen Schwefelverbindungen (TOS) des Partikels größer ist als 87 Massen-%, insbesondere größer als 88 Massen-%, 89 Massen-% oder 90 Massen-%, im Verhältnis zur Gesamtmasse des Partikels,
wobei das Verfahren umfasst: einen Schritt der Pulverisierung, auf einem Feststoff, im Wesentlichen bestehend aus einem Salz mit der Formel (I), wie im Vorstehenden definiert, einer Zusammensetzung, die eine Verbindung B umfasst, ausgewählt aus der Gruppe bestehend aus 2-Hydroxy-4-methylthiobutansäure (HMTBA), wobei die Masse der Verbindung B von 10 % bis 50 % der Masse des Salzes mit der Formel (I) des Feststoffs beträgt,
um das Partikel zu erhalten,
wobei die Zusammensetzung insbesondere außerdem Wasser umfasst, wobei der Massenprozentsatz von Wasser der Zusammensetzung von 0,5 bis 50 beträgt.

**11.** Verfahren nach Anspruch 10, wobei der Schritt der Pulverisierung durchgeführt wird:

- diskontinuierlich oder kontinuierlich in einem Luftwirbelbett, oder
- in einem Vibrofluidisator, oder
- in einem Zerstäubungsturm durch Copulverisierung,

wobei der Schritt der Pulverisierung insbesondere kontinuierlich vorgenommen wird.

**12.** Verfahren nach einem der Ansprüche 10 bis 11, wobei der Kern, der im Wesentlichen aus einem Salz mit der Formel (I) besteht, erhalten wird:

- durch reaktive Zerstäubung,
- in einem Luftwirbelbett, in einem Granulator, in einem Rotationsgranulator oder in einem Mischer,
- durch reaktive Extrusion, oder
- mit Hilfe eines statischen oder dynamischen Mischers.

**Claims**

**1.** A particle comprising:

• a core consisting essentially of a salt of formula (I) below:

$$(A^-)_n M^{n+} \qquad (I)$$

in which:

A⁻ represents an anion chosen from the group consisting of 2-hydroxy-4-methylthiobutanoate, methioniate and cysteinate,
M represents a divalent or trivalent metal,
n being equal to 2 when said metal is divalent and to 3 when said metal is trivalent, and

• a layer comprising a compound B chosen from the group consisting of 2-hydroxy-4-methylthiobutanoic acid (HMTBA), salts thereof, complexes thereof and mixtures thereof,

said layer coating said core,
the weight percentage of said compound B relative to the salt of formula (I) of the core being comprised from 10% to 40%,
said compound B not being, or not only being, in the form of a salt of formula (I),
the organosulfur compound content (TOS) of said particle being greater than 87% by weight, in particular greater than 88%, 89% or 90% by weight, relative to the total weight of said particle.

2. A particle according to claim 1, wherein said compound B is in the:

- free form, chosen from 2-hydroxy-4-methylthiobutanoic acid (HMTBA), and/or
- form of salt of said formula (I) as defined in claim 1, and/or
- form of a complex of formula $(A)_4M$ (II) in which A and M are as defined in claim 1, A representing 2-hydroxy-4-methylthiobutanoic acid (HMTBA),

said compound B not being, or not only being, in the form of a salt of formula (I),
said compound B being in particular in the:

- free form,
- form of the complex of formula (II),
- form of a mixture of the free form and of the complex of formula (II),
- form of a mixture of the free form and of the salt of formula (I),
- form of a mixture of the salt of formula (I) and of the complex of formula (II), or
- form of a mixture of the free form, of the salt of formula (I) and of the complex of formula (II).

3. A particle according to any one of claims 1 to 2, wherein:

- the water content is less than 3%, in particular less than 2% or 1.5%, by weight,
- the calcium content is comprised from 6% to 11% by weight, in particular from 6.5% to 10%, particularly from 7% to 9%, the calcium content being in particular 8%, or
- the weight percentage of said compound B relative to the salt of formula (I) of the core is comprised from 10% to 40%, in particular from 15% to 35%, particularly from 20% to 32%.

4. A particle according to any one of claims 1 to 3, wherein said metal is chosen from the group comprising Mg, Be, Ca, Sr, Ba, Mn, Fe, Co, Ni, Cu, Zn, Pt, B, Al, Ga, In, in particular Mg, Ca, Fe, Mn, Al, Cu, Zn, said salt of formula (I) being in particular $(HMTBA)_2Ca$, $(HMTBA)_2Mg$, $(HMTBA)_2Fe$, $(HMTBA)_2Mn$, $(HMTBA)_2Zn$, $(HMTBA)_2Cu$, $(HMTBA)_3Fe$, $(HMTBA)_3Al$, $(Methionine)_2Ca$, $(Methionine)_2Mg$, $(Methionine)_2Fe$, $(Methionine)_2Mn$, $(Methionine)_2Zn$, $(Methionine)_2Cu$, $(Methionine)_3Fe$, $(Methionine)_3Al$, $(Cysteine)_2Ca$, $(Cysteine)_2Mg$, $(Cysteine)_2Fe$, $(Cysteine)_2Mn$, $(Cysteine)_2Zn$, $(Cysteine)_2Cu$, $(Cysteine)_3Fe$, or $(Cysteine)_3Al$, particularly a salt of formula $(HMTBA)_2Ca$, $(HMTBA)_2Mg$, $(HMTBA)_2Fe$, $(HMTBA)_2Mn$, $(HMTBA)_2Zn$, $(HMTBA)_2Cu$, $(Methionine)_2Ca$, $(Methionine)_2Mg$, $(Methionine)_2Fe$, $(Methionine)_2Mn$, $(Methionine)_2Zn$, $(Methionine)_2Cu$, $(Cysteine)_2Ca$, $(Cysteine)_2Mg$, $(Cysteine)_2Fe$, $(Cysteine)_2Mn$, $(Cysteine)_2Zn$ or $(Cysteine)_2Cu$.

5. A particle according to any one of claims 1 to 3, wherein:

- said anion $A^-$ is 2-hydroxy-4-methylthiobutanoate,
and/or
- said compound B included in said layer is 2-hydroxy-4-methylthiobutanoic acid (HMTBA), or a salt or complex thereof.

6. A pulverulent composition consisting of or comprising particles according to any one of claims 1 to 5.

7. A pulverulent composition of particles according to claim 6, wherein the particle size of said particles ranges from 10 to 3000 $\mu$m, in particular from 20 to 300 $\mu$m, particularly from 100 to 250 $\mu$m, in terms of mean particle size [Dv(0,5)].

8. A pulverulent composition according to any one of claims 6 to 7, in which:

- the bulk density is greater than 350 g/L, in particular greater than 400 g/L, or
- the tapped density is greater than 400 g/L, in particular greater than 450 g/L.

9. A pulverulent composition according to any one of claims 6 to 8, comprising, in addition to said particles, oil, in particular vegetable oil.

10. A process for producing a particle comprising:

• a core consisting essentially of a salt of formula (I) below:

$$(A^-)_n M^{n+} \qquad (I)$$

in which:

$A^-$ represents an anion chosen from the group consisting of 2-hydroxy-4-methylthiobutanoate, methioniate and cysteinate,
M represents a divalent or trivalent metal,
n being equal to 2 when said metal is divalent and to 3 when said metal is trivalent, and

• a layer comprising a compound B chosen from the group consisting of 2-hydroxy-4-methylthiobutanoic acid (HMTBA),

said layer coating said core,
the weight percentage of said compound B relative to the salt of formula (I) of the core being comprised from 10% to 50%,
said compound B not being, or not only being, in the form of a salt of formula (I),
the organosulfur compound content (TOS) of said particle being greater than 87% by weight, in particular greater than 88%, 89% or 90% by weight, relative to the total weight of said particle,
said process comprising a step of spraying, onto a solid consisting essentially of a salt of formula (I) as defined above, a composition comprising a compound B chosen from the group consisting of 2-hydroxy-4-methylthiobutanoic acid (HMTBA), the weight of said compound B being comprised from 10% to 50% of the weight of the salt of formula (I) of the solid,
in order to obtain said particle,
in particular, said composition also comprising water, the weight percentage of said water of said composition being comprised from 0.5 to 50.

11. A process according to claim 10, wherein said spraying step is carried out:

- batchwise or continuously in a fluidized airbed,
or
- on a vibro-fluidizer,
or
- in a spray tower by co-spraying,

said spraying step being in particular carried out continuously.

12. A process according to any one of claims 10 to 11, wherein the core consisting essentially of a salt of formula (I) is obtained:

- by reactive atomization,
- in a fluidized airbed, in a granulator, in a rotary granulator, or in a mixer,
- by a reactive extrusion, or
- by means of a static or dynamic mixer.

**FIGURE 1**

A                                    B

**FIGURE 2**

A                                    B

**FIGURE 3A**

**FIGURE 3B**

## FIGURE 3C

**FIGURE 4**

B

A

# FIGURE 5

**FIGURE 6**

**FIGURE 7**

FIGURE 8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4335257 A
- US 3272860 A
- EP 0049057 A
- US 6287627 B
- FR 2964968
- EP 140865 A
- FR 2988091

**Littérature non-brevet citée dans la description**

- **ROMOSER et al.** *Poult. Sci.,* 1976, vol. 55 (3), 1099-1103